# EUROPEAN PATENT APPLICATION

(11) **EP 4 785 983 A1**
(43) Date of publication of application: **05.08.2026**
(21) Application number: 24872336.3
(22) Date of filing: 26.09.2024
(51) Int. Cl.: A61M 25/10, A61M 25/09

(54) **BALLOON-EQUIPPED GUIDE WIRE, CATHETER SET, AND MEDICAL INSTRUMENT**

(30) Priority: 27.09.2023 JP 2023165927; 27.09.2023 JP 2023165928
(71) Applicant: SB-Kawasumi Laboratories, Inc., Kawasaki-shi, Kanagawa 210-8602 (JP); Sumitomo Bakelite Co.Ltd., Shinagawa-ku Tokyo 140-0002 (JP)
(72) Inventor: FUJITA, Yasuhiro, Kawasaki-shi, Kanagawa 210-8602 (JP); YAMAGUCHI, Kenjiro, Kawasaki-shi, Kanagawa 210-8602 (JP); SHIBATA, Kohei, Kawasaki-shi, Kanagawa 210-8602 (JP); KATAYAMA, Takahiro, Kawasaki-shi, Kanagawa 210-8602 (JP)
(74) Representative: Müller-Boré & Partner Patentanwälte PartG mbB
(86) International application number: PCT/JP2024/034314
(87) International publication number: WO 2025/070550

(57) **Abstract**

A portion of a tube wall of a tubular member (10) on a leading end side is a spring. In the spring, a ribbon (15a) is helically arranged with a gap between turns with a central axis of the tubular member (10) as an axis. A width dimension of a helical slit (44) is less than one-fifth of a width dimension of the ribbon (15a). When a fluid is injected through an injection port (18) to increase a volume of the balloon member (40), the balloon member (40) is expanded by increasing dimensions of the balloon member (40) in both a radial direction and an axial direction of the tubular member (10).

## Description

### TECHNICAL FIELD

The present invention relates to a balloon-equipped guide wire, a catheter set including such a balloon-equipped guide wire, and a medical instrument.

### BACKGROUND ART

There is a guide wire that is inserted into a body lumen such as a blood vessel and has, at a leading end portion, a balloon for occluding the body lumen. Regarding this kind of technology, in Patent Document 1, a balloon member (16) is provided at a distal end portion (leading end portion) of a tubular member (14) that is a hypotube. As shown in FIG. 2 of Patent Document 1, the balloon member (16) is coaxially attached to a distal end (14B) of the tubular member (14) at a proximal end (16A) and a distal end (16B) of the balloon member (16) by appropriate adhesives (19) and (21).

### Citation List

### Patent Document

Patent Document 1: Japanese Unexamined Patent Application No. 2001-514544

### SUMMARY OF INVENTION

### Technical Problem

In Patent Document 1, inside the balloon member (16), the tubular member (14) that is a hypotube made of a nitinol alloy is arranged, and the proximal end (16A) and the distal end (16B) of the balloon member (16) are each fixed to the tubular member (14). Accordingly, the balloon member (16) expands in a radial direction of the tubular member (14) to a spherical shape.

In this case, although a largest-diameter portion of the balloon member (16), which has been expanded to a spherical shape, is pressed against an inner wall of a body lumen such as a blood vessel, a range, in an axial direction of the tubular member (14), of the balloon member (16) that is pressed against the inner wall is small, and thus occlusion of the body lumen may be undesirable, or an unnecessary pressure may be applied to the inner wall of the body lumen.

The present invention has been made in view of the above problems, and provides a balloon-equipped guide wire that achieves improved occlusion of a body lumen and suppresses application of an unnecessary pressure to an inner wall of the body lumen. Solution to Problem

The present invention relates to a balloon-equipped guide wire including: a tubular member that has a lumen and is provided with an opening through which the lumen communicates with an exterior space in a tube wall at a base end portion of the tubular member; a balloon member that is provided on an outer diameter side of a leading end portion of the tubular member, has an inner portion that communicates with the lumen of the tubular member, and is expandable by increasing a volume of the inner portion; and a linear member that is arranged in the lumen at the base end portion of the tubular member and is movable in an axial direction of the tubular member, in which a sealing member that is in close contact with a portion of an inner wall of the tubular member is provided at a leading end portion of the linear member, the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening, the tube wall at the leading end portion of the tubular member is a spring in which a ribbon is helically arranged with a gap between turns with a central axis of the tubular member as an axis, a width dimension of the gap is less than 1/5 of a width dimension of the ribbon, and when a liquid or a gas is injected through the opening to increase the volume of the balloon member, the balloon member is expanded by increasing dimensions of the balloon member in both a radial direction and the axial direction of the tubular member.

The present invention relates to a catheter set including: a balloon-equipped guide wire; and a catheter into which the balloon-equipped guide wire is inserted, in which the balloon-equipped guide wire includes a tubular member that has a lumen and is provided with an opening through which the lumen communicates with an exterior space in a tube wall at a base end portion of the tubular member, a balloon member that is provided on an outer diameter side of a leading end portion of the tubular member, has an inner portion that communicates with the lumen of the tubular member, and is expandable by increasing a volume of the inner portion, and a linear member that is arranged in the lumen at the base end portion of the tubular member and is movable in an axial direction of the tubular member, a sealing member that is in close contact with a portion of an inner wall of the tubular member is provided at a leading end portion of the linear member, the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening, the tube wall at the leading end portion of the tubular member is a spring in which a ribbon is helically arranged with a gap between turns with a central axis of the tubular member as an axis, a width dimension of the gap is less than 1/5 of a width dimension of the ribbon, and when a liquid or a gas is injected through the opening to increase the volume of the balloon member, the balloon member is expanded by increasing dimensions of the balloon member in both a radial direction and the axial direction of the tubular member.

As described above, the balloon member is expanded in the dimension in the axial direction of the tubular member, so that the range of the balloon member in pressure contact with the inner wall of the body lumen is increased in the axial direction of the tubular member. As a result, the body lumen is more favorably occluded. Alternatively, a surface area of the balloon member in pressure contact with the inner wall of the body lumen is increased, so that the pressure applied from the balloon member to the inner wall of the body lumen is reduced, and unnecessary pressure is prevented from being applied to the inner wall of the body lumen.

### Advantageous Effects of Invention

With the balloon-equipped guide wire according to the present invention, the occlusion of the body lumen is more favorable or unnecessary pressure is prevented from being applied to the inner wall of the body lumen.

### BRIEF DESCRIPTION OF DRAWINGS

The above object and other objects, features, and advantages will be further clarified by the following suitable embodiments and the accompanying drawings.
[FIG. 1] A schematic view showing an example of a balloon-equipped guide wire and a catheter according to a first embodiment of the present invention.
[FIG. 2] (a) of FIG. 2 and (b) of FIG. 2 are partially enlarged schematic views of a frame II shown by a dashed line in FIG. 1. (a) of FIG. 2 shows an example of a case where a sealing member is at an open position, and (b) of FIG. 2 shows an example of a case where the sealing member is at a closed position.
[FIG. 3] A schematic view showing a structure of a linear member according to the first embodiment.
[FIG. 4] An enlarged schematic view of a frame IV shown by a dashed line in FIG. 1.
[FIG. 5] A schematic view showing an example of a balloon member and the vicinity thereof according to the first embodiment.
[FIG. 6] (a) of FIG. 6 to (c) of FIG. 6 are explanatory views showing a process of expansion of the balloon member according to the present embodiment. (a) of FIG. 6 is a schematic view of the balloon member and the vicinity thereof in a reduced-diameter state, (b) of FIG. 6 is a schematic view of the balloon member and the vicinity thereof in a small-diameter state, and (c) of FIG. 6 is a schematic view of the balloon member and the vicinity thereof in a large-diameter state.
[FIG. 7] A graph showing a relationship between an amount of injected liquid and a balloon size.
[FIG. 8] A graph showing a relationship between a clearance between an inner diameter of a tubular member according to a second embodiment of the present invention and an amplitude of a linear member in a natural state and a pull-out strength.

### DESCRIPTION OF EMBODIMENTS

Various components of a balloon-equipped guide wire, a catheter set, and a medical instrument according to the present invention do not need to be independent of each other, and it is allowed that a plurality of components are formed as one member, one component is formed of a plurality of members, a certain component is a portion of other components, or a portion of a certain component and a portion of other components overlap with each other.

Hereinafter, embodiments of the present invention will be described with reference to the accompanying drawings. In each drawing, the same reference numerals are given to corresponding components, and duplicated descriptions will be omitted as appropriate.

The term "plane" in the present invention means a shape that is physically formed as a target plane, and it is not necessary to be a geometrically perfect plane.

### <First Embodiment>

### (Balloon-Equipped Guide Wire)

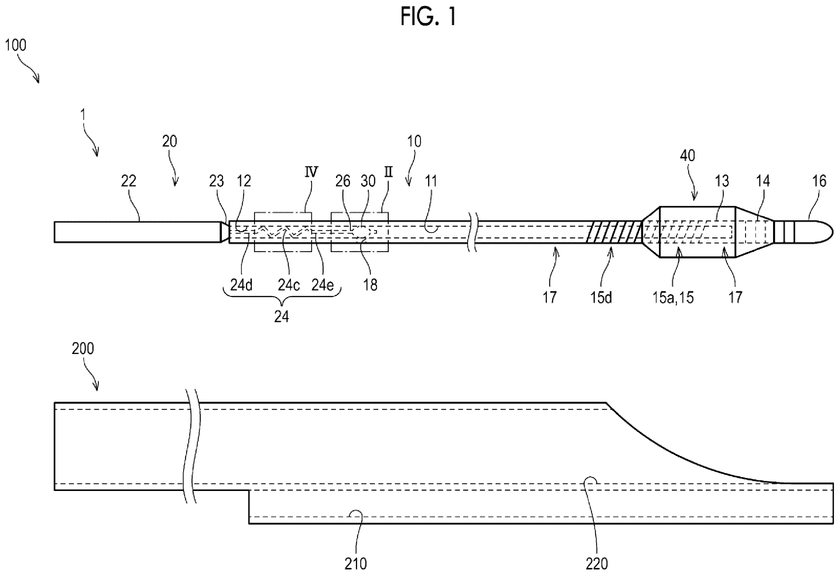

FIG. 1 is a schematic view showing an example of a balloon-equipped guide wire 1 according to a first embodiment of the present invention.

First, an overview of the balloon-equipped guide wire 1 according to the present embodiment will be described.

The balloon-equipped guide wire 1 includes a tubular member 10, a balloon member 40, and a linear member 20.

The tubular member 10 has a lumen 11. An opening (injection port 18) is provided in a tube wall that forms a portion (injection port forming portion) of the tubular member 10 on a base end side. The lumen 11 communicates with an exterior space through the injection port 18.

The balloon member 40 is provided on an outer diameter side of a portion (attachment portion 13 described later) of the tubular member 10 on a leading end side. An inner portion (hollow portion 42) of the balloon member 40 communicates with the lumen 11 of the tubular member 10. The balloon member 40 is expandable by increasing a volume of the hollow portion 42.

The linear member 20 is arranged in a portion of the lumen 11 of the tubular member 10 on the base end side. The linear member 20 is movable in an axial direction of the tubular member 10. A sealing member 30 is provided at a leading end portion 26 of the linear member 20. The sealing member 30 is in close contact with a portion of an inner wall (wall portion that defines the lumen 11) of the tubular member 10.

The sealing member 30 moves between a closed position and an open position with the movement of the linear member 20 in the axial direction. At the closed position, the sealing member 30 closes the injection port 18 or is arranged on the leading end side (distal side) with respect to the injection port 18. In addition, at the closed position, the sealing member 30 blocks a flow of a fluid from the injection port 18 toward the hollow portion 42. The blocking of the flow of the fluid means that the flow of the fluid is sufficiently restricted to a degree that an expanded-diameter state of the balloon member 40 can be maintained for a predetermined time (the fluid is retained), and is not limited to a case where the flow of the fluid is completely blocked. At the closed position, a portion (a portion on the leading end side with respect to the sealing member 30) of the lumen 11 and the hollow portion 42 are liquid-tightly or airtightly sealed. At the open position, the sealing member 30 is positioned on the base end side of the tubular member 10 with respect to the injection port 18.

A portion of the tube wall of the tubular member 10 on the leading end side is a spring (spring forming portion 15). Specifically, in the spring, a ribbon 15a is helically arranged with a gap (helical slit 44) between turns with a central axis of the tubular member 10 as an axis.

A width dimension of the helical slit 44 is less than one-fifth of a width dimension of the ribbon 15a.

When a fluid (a liquid or a gas; referred to as a liquid or the like) is injected through the injection port 18 to increase the volume of the balloon member 40, the balloon member 40 is expanded by increasing the dimensions of the balloon member 40 in both the radial direction and the axial direction of the tubular member 10. As the liquid or the like, a liquid such as physiological saline is preferably used. As the liquid or the like, a mixture containing a contrast agent and physiological saline may be used.

As described above, the balloon member 40 is expanded in the dimension in the axial direction of the tubular member 10, so that the range of the balloon member 40 in pressure contact with the inner wall of the body lumen is increased in the axial direction of the tubular member 10. As a result, the body lumen is more favorably occluded. Alternatively, a surface area of an outer peripheral surface of the balloon member 40 in pressure contact with the inner wall of the body lumen is increased, so that the pressure applied from the balloon member 40 to the inner wall of the body lumen is reduced, and unnecessary pressure is prevented from being applied to the inner wall of the body lumen. Further, since the balloon member 40 can be expanded in the axial direction, the amount of fluid that can be injected into the hollow portion 42 is increased as compared with a case where the balloon member 40 is expanded only in the radial direction. Therefore, for example, even when an excessive amount of fluid is unexpectedly injected into the balloon member 40, the balloon member 40 is prevented from being damaged due to the balloon member 40 expanded in the axial direction.

In addition, since the width dimension of the helical slit 44 is a small value less than one-fifth of the width dimension of the ribbon 15a, the helical direction of the ribbon 15a with respect to the axial direction of the tubular member 10 is further increased. As a result, the portion in which the spring is formed is easily expanded and contracted in the axial direction of the tubular member 10. In addition, since the width dimension of the helical slit 44 is a small value less than one-fifth of the width dimension of the ribbon, and the spring is tightly wound, the portion in which the spring is formed has a large room to be expanded in the axial direction of the tubular member 10.

Next, the balloon-equipped guide wire 1 (medical instrument 1) according to the present embodiment will be described in detail.

The balloon-equipped guide wire 1 is a member that is inserted into the body lumen in the living body, and is an elongated member as a whole. For the members such as the balloon-equipped guide wire 1, the tubular member 10, and the linear member 20, one end that is first inserted into the body lumen in a longitudinal direction (axial direction) of the balloon-equipped guide wire 1 may be referred to as a leading end, and the other end (one end on a hand side of an operator) opposite to the leading end may be referred to as a base end. In addition, the base end side may be referred to as a hand side or a proximal side, and a side opposite to the proximal side may be referred to as a distal side.

The balloon-equipped guide wire 1 according to the present embodiment may be used together with other devices such as a catheter 200 which will be described in a second embodiment, or may be used alone.

The tubular member 10 is an elongated member that extends in the longitudinal direction of the balloon-equipped guide wire 1 as the axial direction. The axial direction of the tubular member 10 may be simply referred to as the axial direction. In the present embodiment, the tubular member 10 has superelasticity. For example, examples of the material forming the tubular member 10 include a metal such as nickel titanium (nitinol). Alternatively, the tubular member 10 may be made of another material such as an elastic resin. Alternatively, the tubular member 10 may be made of a material in which a resin and a metal are mixed, or may be formed by combining a resin portion and a metal portion.

The tubular member 10 has the lumen 11 inside. A cross-sectional shape of the tubular member 10 is a circle, a polygon, or the like. A width (inner diameter of the tubular member 10) of the lumen 11 is larger than a wire diameter of the linear member 20 described later. A lower limit of the outer diameter of the tubular member 10 is 250 µm and preferably 300 µm, and an upper limit of the outer diameter of the tubular member 10 is 400 µm and preferably 350 µm. A lower limit of the inner diameter of the tubular member 10 is 150 µm and preferably 200 µm, and an upper limit of the inner diameter of the tubular member 10 is 300 µm and preferably 250 µm. A lower limit of a thickness dimension of the tube wall of the tubular member 10 is 50 µm and preferably 80 µm, and an upper limit of the thickness dimension of the tube wall of the tubular member 10 is 150 µm and preferably 120 µm.

In the tubular member 10 according to the present embodiment, the injection port 18 (through-hole) that is the opening through which the lumen 11 communicates with an exterior space, and is provided in a portion (a portion into which the linear member 20 described later can be inserted) of the tube wall on the base end side. The injection port 18 is provided to inject a solution or a gas for expanding the balloon member 40 or provided to inject a drug. The solution or the like can be injected into the lumen 11 through the injection port 18.

A shape of the injection port 18 when viewed from the side of the balloon-equipped guide wire 1 may be circular or polygonal.

A dimension of the injection port 18 in a predetermined direction in plan view (particularly, a dimension of the injection port 18 in the axial direction) of the injection port 18 according to the present embodiment is smaller than a length of the sealing member 30 in the axial direction. A diameter of the injection port 18 is preferably larger than the thickness dimension of the tube wall of the tubular member 10.

As shown in FIG. 1, the lumen 11 in the present embodiment is closed at a leading end portion (a portion on the base end side with respect to a coil portion described later).

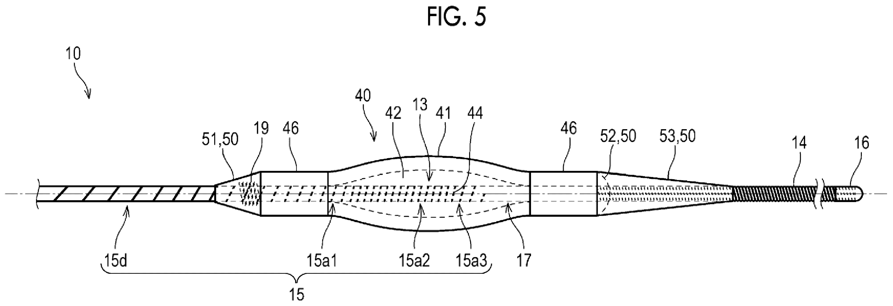

In addition, as shown in FIG. 5, in order to increase the flexibility of the tubular member 10, a portion (coil portion 14) of the tubular member 10 on the most leading end side is formed of a coil in which a wire is tightly helically wound. Further, in order to facilitate the insertion of the tubular member 10 into the body lumen, a leading end tip 16 is fitted to a leading end of the coil portion 14. The leading end tip 16 in the present embodiment is made of a metal such as an alloy of tin and gold.

The balloon member 40 is a member that is arranged in a portion of the body lumen of the living body, is in pressure contact with the wall portion that defines the body lumen by being expanded to seal the body lumen, or is used to close the opening provided in the wall portion. The balloon member 40 does not need to completely seal the body lumen or completely close the opening of the wall portion, and it is sufficient to dam the body lumen to a degree that the flow of the liquid or the like in the body lumen or the opening of the wall portion can be restricted, or it is sufficient to cover the opening of the wall portion. The balloon member 40 is arranged in the blood vessel in the expanded-diameter state and is in pressure contact with a blood vessel wall (inner wall of the blood vessel) to suppress the flow of blood.

As shown in FIG. 5, the balloon member 40 has the hollow portion 42 covered by a film portion 41 inside. The hollow portion 42 of the balloon member 40 communicates with the lumen 11 (see FIG. 1) of the tubular member 10. For example, a through-hole portion such as a groove through which the lumen 11 communicates with the exterior space is provided in a portion of the tube wall of the tubular member 10 arranged on the inner diameter side of the balloon member 40, and the hollow portion 42 and the lumen 11 communicate with each other through the through-hole portion. In the present embodiment, as will be described later, the helical slit 44 is provided in the tube wall of the tubular member 10, and the hollow portion 42 and the lumen 11 communicate with each other through the helical slit 44 that is the through-hole portion. By injecting a liquid or a gas (referred to as a liquid or the like) into the hollow portion 42 of the balloon member 40 through the injection port 18, the lumen 11, and the helical slit 44, the volume of the hollow portion 42 is increased and the balloon member 40 is expanded in diameter. The balloon member 40 being expanded in diameter means that the dimension of the balloon member 40 in the radial direction of the balloon-equipped guide wire 1 is increased as compared with before the injection of the liquid or the like. The radial direction of the balloon-equipped guide wire 1 is a radial direction from an axial center (axial center of the tubular member 10) of the balloon-equipped guide wire 1 toward a peripheral edge (peripheral edge of the tubular member 10) of the balloon-equipped guide wire 1 when viewed in the axial direction.

As shown in FIG. 5, the spring forming portion 15 that is a portion of the tubular member 10 on the leading end side is a spring that is expandable and contractible in the axial direction by forming a slit (helical slit 44) in a helical shape. Specifically, the tube wall remaining by forming the helical slit 44 is a thin ribbon 15a, and the ribbon 15a is helically arranged over a plurality of turns to form a spring.

In the present embodiment, the ribbon 15a is a plate-shaped portion having a predetermined thickness. The ribbon 15a has a predetermined width dimension. The width dimension of the ribbon 15a is a distance between one end on the leading end side and one end on the base end side of the ribbon 15a in the axial direction.

In this way, the width dimension of the helical slit 44 is less than one-fifth of the width dimension of the ribbon 15a, but more preferably the width dimension of the helical slit 44 is less than one-sixth of the width dimension of the ribbon 15a. In addition, in order to stably inject the liquid or the like into the hollow portion 42, the width dimension of the helical slit 44 is preferably equal to or more than one-tenth of the width dimension of the ribbon 15a and more preferably one-twentieth of the width dimension of the ribbon 15a. The width dimension of the helical slit 44 is a distance between one end on the leading end side of one turn of the ribbon 15a and one end on the base end side of another turn adjacent to the one turn in the axial direction. In the present embodiment, the width dimension of the helical slit 44 is constant throughout the spring forming portion 15.

Here, the ribbon 15a width being equal to or more than the predetermined value or equal to or less than (less than) the predetermined value refers to the width of the narrowest ribbon 15a (ribbon 15a in a second region 15a2 described later) in the entire spring forming portion 15. That is, the ribbon 15a in the length region other than the second region 15a2 need not correspond to a value equal to or more than the predetermined value or equal to or less than (less than) the predetermined value. Alternatively, the ribbon 15a width of the entire spring forming portion 15 may be a value equal to or more than the predetermined value or equal to or less than (less than) the predetermined value.

In the present embodiment, the helical direction of the ribbon 15a is opposite to the helical direction in the coil portion 14. In addition, the helical direction of the ribbon 15a is opposite to a helical direction of a radiopaque marker 19 described later. As a result, the radiopaque marker 19 is prevented from interfering with the helical slit 44 of the spring forming portion 15. Alternatively, the helical direction of the ribbon 15a may be the same as the helical direction in the coil portion 14 or the radiopaque marker 19.

As shown in FIG. 5, the balloon member 40 (particularly, the film portion 41 that is a body portion) is arranged to surround the tubular member 10 (particularly, the attachment portion 13) and has a cylindrical shape. A seal band 46 is arranged on an outer layer side of a portion of the balloon member 40 on the leading end side and a portion of the balloon member 40 on the base end side. The seal band 46 presses the balloon member 40 from the outer layer side to assist in fixing the balloon member 40 to the tubular member 10.

In the present embodiment, a resin layer (not shown) that serves as a base for arranging the balloon member 40 is provided on the outer layer side of the tubular member 10. The resin layer may be made of a resin such as polyethylene terephthalate (PET). The resin layer is arranged, for example, on a portion of the tubular member 10 on which the seal band 46 is arranged and on the outer layer side of a base end side forming portion 15d described later. The resin layer is not arranged on the outer layer side of a portion of the tubular member 10 (second region 15a2 or the like) arranged in the hollow portion 42. As a result, the hollow portion 42 and the lumen 11 communicate with each other.

In addition, the balloon member 40 (particularly, the film portion 41) is fixed (bonded) to the tubular member 10 by an appropriate adhesive 50. More specifically, a first adhesive 51 is arranged on the outer layer side of the tubular member 10 on the base end side with respect to the balloon member 40. The first adhesive 51 is formed to have a tapered shape. In the present embodiment, a coil-shaped radiopaque marker 19 is embedded in the first adhesive 51. That is, the first adhesive 51 may be arranged on the inner diameter side and the outer diameter side of the radiopaque marker 19, or the first adhesive 51 may be arranged between the turns of the radiopaque marker 19. In addition, a second adhesive 52 is provided in a dome shape that bulges toward the leading end side of the balloon member 40. The third adhesive 53 is formed on the outer layer side of the tubular member 10 in a tapered shape to cover the second adhesive 52. The third adhesive 53 is arranged on the outer layer side of the coil portion 14.

In the present embodiment, the thickness (width dimension) of the adhesive 50 is the same as the thickness (particularly, the thickness of the seal band 46) of the balloon member 40. A length of the first adhesive 51 in the axial direction is smaller than a length of the third adhesive 53 in the axial direction. An angle formed by the tapered shape of the third adhesive 53 with respect to the axial direction is smaller than an angle formed by the tapered shape of the first adhesive 51 with respect to the axial direction. As a result, the flexibility of the coil portion 14 is less likely to be hindered by the third adhesive 53. In addition, the insertion of the balloon-equipped guide wire 1 into the body lumen (particularly, a stenotic portion) is prevented from being hindered by the third adhesive 53.

As shown in FIG. 1, the linear member 20 is arranged in the lumen 11 of the tubular member 10. Specifically, an opening (base end opening portion 12) that penetrates the tubular member 10 in the axial direction to allow the exterior space of the tubular member 10 and the lumen to communicate with each other is provided at the base end of the tubular member 10. The linear member 20 is inserted into the lumen 11 from the base end opening portion 12. The linear member 20 is arranged in a portion of the tubular member 10 on the base end side. More specifically, the linear member 20 is inserted from the base end of the tubular member 10 to the vicinity of the injection port 18.

In addition, in the present embodiment, a portion (a base end portion 22 and a portion of a second transition portion 23 described later) of the linear member 20 on the base end side protrudes from the base end opening portion 12 at the base end of the tubular member 10 to the base end side and is arranged outside the tubular member 10. Therefore, the linear member 20 can be moved in the axial direction of the tubular member 10 inside the tubular member 10 by gripping a portion (particularly, the base end portion 22) of the linear member 20 arranged outside the tubular member 10 with an instrument or a hand and then pulling out or inserting the linear member 20. By moving the linear member 20 in the axial direction of the tubular member 10, the position of the sealing member 30 attached to the leading end portion 26 of the linear member 20 can be made variable in the axial direction of the tubular member 10. As a result, the inflow and outflow of the liquid or the like in the lumen 11 can be adjusted as will be described later.

The sealing member 30 is a member for adjusting the flow of the liquid or the gas in the lumen 11. The sealing member 30 is fixed (bonded) to the vicinity of the leading end portion 26 of the linear member 20. The sealing member 30 in the present embodiment is made of a resin such as a thermoplastic resin. Instead of the present embodiment, the sealing member 30 may be made of a resin other than the thermoplastic resin or may be made of other materials (high-density cotton or the like) having a small passage amount of the liquid or the gas. The sealing member 30 seals the lumen 11 by being in pressure contact with or in close contact with a portion of the inner wall of the tubular member 10 in the axial direction and substantially the entire circumference of the inner wall. Specifically, the sealing member 30 is in pressure contact with at least a portion of the inner wall of the tubular member 10 in the radial direction of the tubular member 10, and preferably in close contact with the inner wall of the tubular member 10. More preferably, the sealing member 30 is in close contact with the inner wall of the tubular member 10 in the entire radial direction of the tubular member 10. That is, the sealing member 30 seals substantially the entire cross section of the lumen 11. As a result, the inflow and outflow of the liquid or the gas in the lumen 11 in the axial direction are restricted via the sealing member 30. A width dimension of the sealing member 30 when the linear member 20 is taken out from the outside of the tubular member 10 (dimension in a direction orthogonal to the axial direction of the linear member 20) is preferably larger than the width of the lumen 11. As a result, the sealing member 30 is in pressure contact with a portion of the inner wall of the tubular member 10 in the lumen 11.

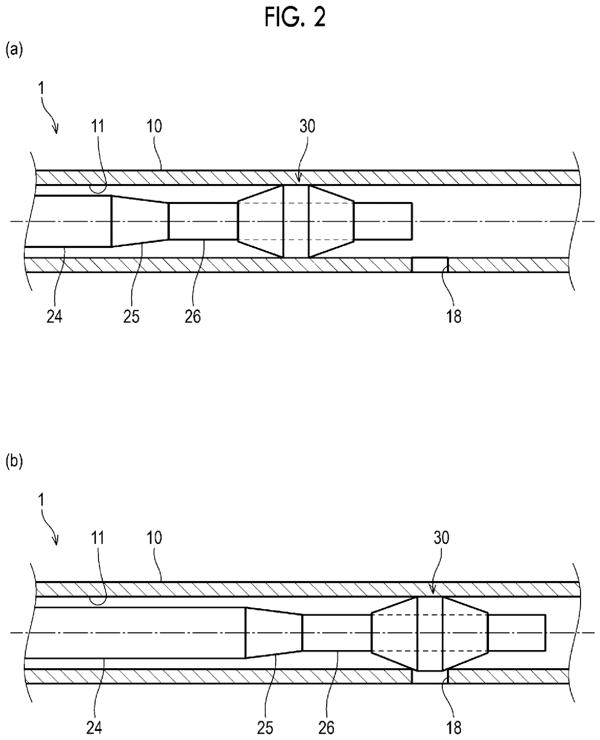

As shown in (a) and (b) of FIG. 2 and described above, the sealing member 30 is movable between the open position and the closed position with the movement of the linear member 20 in the axial direction. The closed position of the sealing member 30 is a position at which the sealing member 30 closes the injection port 18 or is arranged on the leading end side (distal side) with respect to the injection port 18 to liquid-tightly or airtightly seal a portion (a portion on the leading end side with respect to the sealing member 30) of the lumen 11 and the hollow portion 42. That is, at the closed position, the flow of the fluid (liquid or the like) from the injection port 18 toward the hollow portion 42 through the lumen 11 is blocked by the sealing member 30. The blocking of the flow of the fluid means that the flow of the fluid is sufficiently restricted to a degree that an expanded-diameter state of the balloon member 40 can be maintained for a predetermined time (the fluid is retained), and is not limited to a case where the flow of the fluid is completely blocked.

The open position of the sealing member 30 is a position on the base end side of the tubular member 10 with respect to the injection port 18. (a) of FIG. 2 shows a case where the sealing member 30 is at the open position, and (b) of FIG. 2 shows a case where the sealing member 30 is at the closed position. When the sealing member 30 is at the closed position or the open position, the position of the other member may be referred to as the closed position or the open position.

As shown in (a) of FIG. 2, at the open position, the sealing member 30 is arranged at a position on the base end side with respect to the injection port 18. Therefore, a portion of the lumen 11 on the leading end side with respect to the injection port 18 and the hollow portion 42 (see FIG. 5) communicate with the exterior space of the tubular member 10 through the injection port 18. Therefore, the liquid or the like can be injected into the hollow portion 42 through the injection port 18, or the liquid or the like in the hollow portion 42 can be pulled out through the injection port 18. In the open state, the inflow of the liquid or the like from the injection port 18 to a portion of the lumen 11 on the base end side is suppressed by the sealing member 30.

As shown in (b) of FIG. 2, at the closed position, the sealing member 30 is arranged at substantially the same position as the injection port 18 or at a position on the leading end side with respect to the position in the axial direction of the tubular member 10. Specifically, when the sealing member 30 is at substantially the same position as the injection port 18, the sealing member 30 and the injection port 18 at least partially overlap with each other when viewed from the side of the tubular member 10. In this case, the sealing member 30 is in contact (in pressure contact) with at least a portion of an edge portion of the injection port 18. It is preferable that, when viewed from the side of the tubular member 10 (in a direction orthogonal to the axial direction), the entire injection port 18 overlaps with the sealing member 30. In this case, it is preferable that the sealing member 30 is in contact (in pressure contact) with substantially the entire edge portion of the injection port 18 (a portion of the tube wall that defines the injection port 18 and is arranged to surround the injection port 18 once around). As a result, at least a portion (preferably, the entire portion) of the injection port 18 is closed by the sealing member 30. As a result, the liquid or the like is prevented from leaking out from the injection port 18. When the liquid or the like is injected into the lumen 11 and the hollow portion 42 to expand the balloon member 40, the liquid or the like in the hollow portion 42 is prevented from leaking to the outside through the lumen 11 and the injection port 18, so that the expanded-diameter state of the balloon member 40 is maintained.

As shown in (b) of FIG. 2, in the sealing member 30 according to the present embodiment, the center in the axial direction is a large-diameter portion, and the diameter is decreased from the large-diameter portion toward both ends in the axial direction. A width of the large-diameter portion of the sealing member 30 is larger than the width of the lumen 11 in a natural state (a state in which the tubular member 10 is arranged outside). In addition, a length of the large-diameter portion in the axial direction is smaller than the dimension of the injection port 18 in the axial direction. Therefore, as shown in FIG. 2, when the sealing member 30 is arranged at the closed position to close the injection port 18, a portion on both ends of the large-diameter portion is in pressure contact with a portion of the tube wall that defines the injection port 18. Instead of the present embodiment, the length of the large-diameter portion in the axial direction may be the same as or larger than the dimension of the injection port 18 in the axial direction. In this case, as shown in (b) of FIG. 2, when the sealing member 30 is arranged at the closed position to close the injection port 18, it is preferable that the large-diameter portion is in pressure contact with a portion of the tube wall that defines the injection port 18.

The linear member 20 includes a leading end portion 26, an intermediate portion 24, and a base end portion 22. The intermediate portion 24 is a portion of the linear member 20 on the base end side with respect to the leading end portion 26. The base end portion 22 is a portion of the linear member 20 on the base end side with respect to the intermediate portion 24. The leading end portion 26, the intermediate portion 24, and the base end portion 22 each have a partial length region having a uniform outer diameter. Further, among the partial length regions of the leading end portion 26, the intermediate portion 24, and the base end portion 22, the partial length region of the base end portion 22 is the thickest, and the partial length region of the leading end portion 26 is the thinnest.

As shown in FIG. 1, the base end portion 22 of the linear member 20 is a portion of the linear member 20 on the base end side. Specifically, the base end portion 22 is a portion of the linear member 20 that protrudes from the base end opening portion 12 to the base end side and is arranged outside the tubular member 10. The leading end portion 26 of the linear member 20 refers to the portion of the linear member 20 to which the sealing member 30 is fixed and a length region in the vicinity thereof. In the present embodiment, the leading end portion 26 is a length region from the leading end of the linear member 20 to a position slightly on the base end side of the position at which the sealing member 30 is fixed. The intermediate portion 24 of the linear member 20 is a partial length region arranged at a position sandwiched between the base end portion 22 and the leading end portion 26 in the linear member 20. It is preferable that the intermediate portion 24 is arranged in the lumen 11 substantially entirely at the closed position. In addition, in the intermediate portion 24, the partial length region on the base end side may protrude from the base end opening portion 12 to the outside of the lumen 11 at the open position.

Each of the base end portion 22, the intermediate portion 24, and the leading end portion 26 has a uniform outer diameter in the partial length region or an entire length region in the axial direction. In the present embodiment, each of the base end portion 22, the intermediate portion 24, and the leading end portion 26 has a uniform outer diameter in the entire length region in the axial direction. Here, the uniform outer diameter means a substantially uniform outer diameter, and need not have a completely uniform outer diameter due to slight unevenness or the like. The outer diameter and the inner diameter (width of the lumen 11) of the tubular member 10, the outer diameter of the linear member 20 (particularly, the outer diameters of the base end portion 22, the intermediate portion 24, the leading end portion 26, and a transition portion described later), the size of the sealing member 30, and the like in FIG. 1 are exaggerated for convenience. The same applies to (a) of FIG. 2 to FIG. 5.

The outer diameter of the base end portion 22 is preferably substantially equal to the outer diameter of the tubular member 10. Specifically, the outer diameter of the base end portion 22 is preferably equal to or more than two-thirds of the outer diameter of the tubular member 10 and less than 1.5 times the outer diameter of the tubular member 10. A lower limit of the outer diameter of the base end portion 22 is, for example, 0.250 mm and preferably 0.300 mm. An upper limit of the outer diameter of the base end portion 22 is, for example, 0.400 mm and preferably 0.350 mm.

An outer diameter of the intermediate portion 24 is less than the width of the lumen 11 (dimension of the lumen 11 in a direction orthogonal to the axial direction; the diameter of the lumen 11), and preferably less than 85% of the width of the lumen 11. In addition, the outer diameter of the intermediate portion 24 is larger than the outer diameter of the leading end portion 26 and smaller than the outer diameter of the base end portion 22. It is preferable that the outer diameter of the intermediate portion 24 is smaller than a difference between the outer diameter of the leading end portion 26 and the outer diameter of the base end portion 22. As a result, since the intermediate portion 24 has sufficient flexibility, the linear member 20 can be pulled out from the tubular member 10 at a predetermined pull-out strength even when a difference between an amplitude of a wave shape of the intermediate portion 24 and the width of the lumen 11 (clearance described later) is large. This will be described in detail later. In addition, since the outer diameter of the base end portion 22 has a large value equal to or larger than a sum of the outer diameter of the leading end portion 26 and the outer diameter of the intermediate portion 24, the base end portion 22 can be easily gripped and pulled out. Further, since the outer diameter of the leading end portion 26 is a small value less than a difference between the outer diameter of the base end portion 22 and the outer diameter of the intermediate portion 24, the sealing member 30 can be formed to be sufficiently thick to be favorably in pressure contact with the inner wall of the tubular member 10, so that the sealing property of the lumen 11 by the sealing member 30 can be improved.

A lower limit of the outer diameter of the intermediate portion 24 is, for example, 0.15 mm and preferably 0.17 mm. An upper limit of the outer diameter of the intermediate portion 24 is, for example, 0.23 mm and preferably 0.20 mm.

The outer diameter of the leading end portion 26 is preferably equal to or more than half of the width of the lumen 11. In the present embodiment, the outer diameter of the leading end portion 26 is substantially the same as the diameter of the injection port 18. Specifically, the outer diameter of the leading end portion 26 is equal to or more than two-thirds of the diameter of the injection port 18 and less than 1.5 times the diameter of the injection port 18. A lower limit of the outer diameter of the leading end portion 26 is, for example, 0.10 mm and preferably 0.12 mm. An upper limit of the outer diameter of the leading end portion 26 is, for example, 0.20 mm and preferably 0.16 mm.

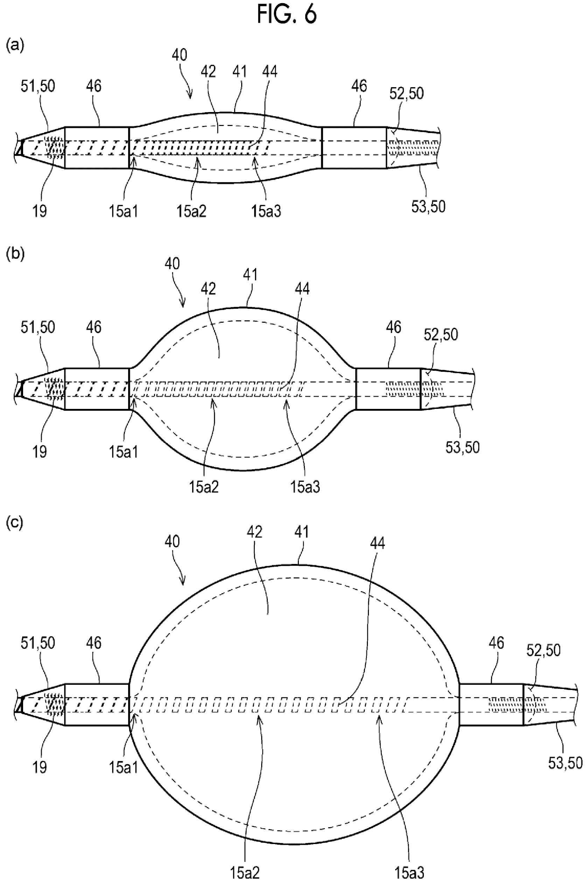

Next, a shape change of the balloon member 40 when the liquid or the like is injected through the injection port 18 to expand the balloon member 40 will be described with reference to (a) to (c) of FIG. 6. As described above, the balloon member 40 is expanded in each of the axial direction and the radial direction by the injection of the liquid or the like into the hollow portion 42. In (a) to (c) of FIG. 6, the helical shape of the spring forming portion 15 is schematically shown. Specifically, the ribbon 15a in the hollow portion 42 is formed in a helical shape, but in (a) to (c) of FIG. 6, the respective turns are divided for convenience and shown to be spaced in the axial direction.

(a) of FIG. 6 shows a reduced-diameter state before the liquid or the like is injected. The reduced-diameter state is a state of the balloon member 40 before the liquid or the like for expanding the balloon member 40 is injected. In the reduced-diameter state, the hollow portion 42 and the lumen 11 may be filled with a fluid (physiological saline or the like). The fluid that fills the hollow portion 42 and the lumen 11 may be the same as or different from the liquid or the like for expanding the balloon member 40. In the reduced-diameter state, the volume of the hollow portion 42 is small or substantially zero. An inner surface of the film portion 41 may be substantially entirely in contact with the tubular member 10 (outer surface of the ribbon 15a in the spring forming portion 15), or may be at least partially spaced from the tubular member 10.

When the fluid for expanding the balloon member 40 is injected (particularly, pressurized) through the injection port 18, the liquid or the like passes through the lumen 11, seeps out from the helical slit 44, and is injected into the hollow portion 42. As a result, the balloon member 40 proceeds from the small-diameter state shown in (b) of FIG. 6 to the large-diameter state shown in (c) of FIG. 6.

Here, the large-diameter state is a state in which the dimension of the balloon member 40 in the width direction is increased to the same degree as the generally assumed maximum inner diameter that can be assumed for the body lumen to be used with the balloon-equipped guide wire 1. For example, in the balloon-equipped guide wire 1 according to the present embodiment, the balloon member 40 is arranged in the blood vessel (a carotid artery is an example), and an example of the general inner diameter of the blood vessel is 5 mm, and an example of the generally assumed large inner diameter of the carotid artery is 6 mm to 7 mm. That is, the large-diameter state of the balloon member 40 in the balloon-equipped guide wire 1 according to the present embodiment is a state in which the balloon member 40 is expanded until the dimension of the balloon member 40 in the width direction becomes about 6.5 mm. Here, the amount of fluid that needs to be pressure-injected from the reduced-diameter state to the large-diameter state is referred to as a total pressure-injection amount. The balloon member 40 may be further expanded in the width direction than the large-diameter state. In the present embodiment, the total pressure-injection amount is about 0.175 ml. The total pressure-injection amount may be equal to or more than 0.160 ml and equal to or less than 0.190 ml, including 0.175 ml. Alternatively, the total pressure-injection amount may be equal to or less than 0.160 ml or equal to or more than 0.190 ml according to the inner diameter of the body lumen. The total pressure-injection amount is about 40% to 50% of the maximum amount of fluid that can be injected into the hollow portion 42 or the like (a limit of the injection amount at which the film portion 41 or the vicinity of the seal band 46 is not damaged).

In addition, here, the small-diameter state is a state of the balloon member 40 when an amount of fluid of about one-fourth of the total pressure-injection amount is injected. In the present embodiment, 0.0425 ml of the fluid is pressure-injected through the injection port 18 from the reduced-diameter state to the small-diameter state. The injection amount is, as an example, about one-tenth of the maximum amount of fluid that can be injected into the hollow portion 42 or the like.

A predetermined amount of fluid is injected from the reduced-diameter state to the small-diameter state. When the fluid is injected, the film portion 41 is pushed in the radial direction by the liquid or the like in the hollow portion 42 to bulge, and the width dimension of the balloon member 40 (dimension in the up-down direction in the drawing) is larger than the width dimension in the reduced-diameter state. Since both ends of the film portion 41 in the axial direction are fixed to the tubular member 10 by the seal band 46, a central portion of the film portion 41 in the axial direction is expanded. Further, by injecting the liquid or the like into the hollow portion 42, the spring formed at the attachment portion 13 is expanded in the axial direction, and the film portion 41 is biased by the liquid or the like to be expanded in the axial direction. When the balloon member 40 is stretched and expanded in the axial direction, a position of the seal band 46 on the base end side may be fixed and the seal band 46 on the leading end side may move toward the leading end side to expand the balloon member 40 in the axial direction.

When the fluid is further injected into the hollow portion 42 from the small-diameter state, as shown in (c) of FIG. 6, the balloon member 40 is further expanded to be in the large-diameter state.

The film portion 41 is biased outward in the radial direction and the axial direction by the liquid or the like in the hollow portion 42, and the balloon member 40 is expanded in the radial direction and the axial direction. In the large-diameter state or in a process of proceeding from the small-diameter state to the large-diameter state, a tension (tension in the axial direction) applied to one end portion of the film portion 41 in the axial direction may be larger than a tension (tension in the axial direction) applied to the center of the film portion 41 in the axial direction (portion having the maximum diameter). In addition, in the large-diameter state, the seal band 46 may be caught in the axial direction at one end portion of the film portion 41 in the axial direction. Specifically, a portion of the seal band 46 may be arranged in the envelope volume of the balloon member 40 (film portion 41) that bulges into a spherical shape.

The balloon member 40 may restrict the blood flow in the body lumen in the small-diameter state or the large-diameter state or an intermediate state thereof. The size of the balloon member 40 in the small-diameter state or the large-diameter state after the expansion may vary depending on the width of the body lumen. For example, the film portion 41 may be suppressed inward in the radial direction by the inner wall that forms the body lumen, and the dimension of the balloon member 40 in the width direction may be smaller than that in a natural state (a state in which the balloon member 40 is not arranged in the body lumen). Further, the dimension of the balloon member 40 in the axial direction may be larger than that in the natural state.

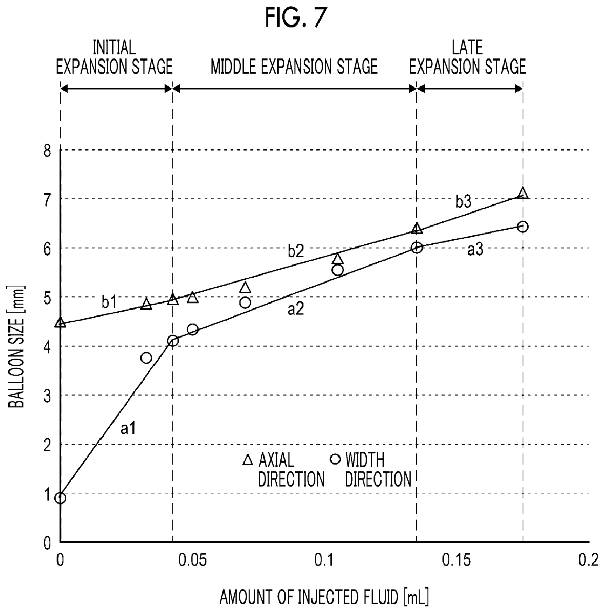

FIG. 7 shows an example of a relationship between the amount of fluid injected through the injection port 18 (the amount of fluid injected with reference to the reduced-diameter state) and the dimension (balloon size) in the axial direction or the width direction. As shown in FIG. 7, the process of expansion from the reduced-diameter state to the large-diameter state is divided into an initial expansion stage, a middle expansion stage, and a late expansion stage. The initial expansion stage is a process of injecting an amount of fluid of about one-fourth of the total pressure-injection amount from the reduced-diameter state to reach the small-diameter state. The middle expansion stage is a process of injecting an amount of fluid of about one-half of the total pressure-injection amount from the small-diameter state to reach the expanded-diameter state (medium-diameter state). The late expansion stage is a process of further injecting an amount of fluid of about one-fourth of the total pressure-injection amount from the medium-diameter state to reach the large-diameter state.

As shown in FIG. 7, in the reduced-diameter state, the dimension in the axial direction is sufficiently large with respect to the dimension in the width direction, but when the fluid is injected (when an amount of fluid of about 0.1 ml is injected), the dimensions in the width direction and the axial direction are substantially equal to each other. That is, in the middle expansion stage, the dimension in the width direction and the dimension in the axial direction are substantially equal to each other. Further, when the fluid is further injected to reach the late expansion stage, the dimension in the axial direction is larger than the dimension in the width direction, and the shape of the balloon member 40 when viewed from the side is an elliptical shape elongated in the axial direction.

**[Table 1]**

| | Expansion rate in width direction (mm/mL) | Expansion rate in axial direction (mm/mL) | Ratio of expansion rate in axial direction to expansion rate in width direction(%) |
|---|---|---|---|
| Initial expansion stage | 75.6 | 10.8 | 14.3 |
| Middle expansion stage | 20.5 | 15.7 | 76.6 |
| Late expansion stage | 10.7 | 18.0 | 168.6 |

Table 1 shows an expansion rate of the dimension in the width direction or the axial direction in each of the initial expansion stage, the middle expansion stage, and the late expansion stage, and a ratio of the expansion rate in the axial direction to the expansion rate in the width direction for the balloon-equipped guide wire 1 according to the present embodiment. The expansion rate of each stage is a value obtained by dividing the amount of change in the dimension during the stage by the amount of fluid injected during the stage. That is, the expansion rate of the dimension in the width direction or the axial direction in each of the initial expansion stage, the middle expansion stage, and the late expansion stage is a slope of a linear function graph in a range shown by a1 to a3 and b1 to b3 in FIG. 7.

The expansion rates in the width direction in the initial expansion stage, the middle expansion stage, and the late expansion stage are respectively 75.6 [mm/ml], 20.5 [mm/ml], and 10.7 [mm/ml]. The expansion rates in the axial direction in the initial expansion stage, the middle expansion stage, and the late expansion stage are respectively 10.8 [mm/ml], 15.7 [mm/ml], and 18.0 [mm/ml].

In the present embodiment, the expansion rate in the width direction decreases as the expansion stage progresses. On the other hand, the expansion rate in the axial direction increases as the expansion stage progresses. That is, in the balloon member 40, the balloon member 40 is not expanded in the axial direction at an initial stage in which the fluid is injected, but is expanded in the axial direction as the fluid is injected and the balloon member 40 is sufficiently expanded. This is because the expandability of the spring in the axial direction is smaller than the expandability of the film portion 41. The expandability is an amount of the member by which the member is expanded in a predetermined direction. Further, in the present embodiment, the expansion rate in the axial direction is smaller than the expansion rate in the width direction in the initial expansion stage, but the expansion rate in the axial direction is larger than the expansion rate in the width direction in the late expansion stage. As described above, in the late expansion stage, the balloon member 40 is expanded in the axial direction, so that the balloon member 40 is arranged to follow the extending direction of the body lumen, and the inner wall of the body lumen and the balloon member 40 are more in contact with each other to favorably seal the body lumen. Further, in the late expansion stage, the balloon member 40 is expanded in the width direction and the axial direction, so that the maximum amount of fluid that can be injected into the hollow portion 42 or the like (the maximum injection amount at which the balloon member 40 is damaged) can be increased. As a result, it is possible to prevent the balloon member 40 from being deformed or damaged even when an excessive amount of fluid is unexpectedly injected.

In FIG. 7 and Table 1, the balloon size when the predetermined amount of fluid is injected in a state in which the balloon member 40 is not particularly restrained from the outer diameter side of the balloon member 40 is shown. In a case where the balloon member 40 is arranged in the body lumen, when the predetermined amount of fluid is injected into the balloon member 40, the outer peripheral surface of the balloon member 40 may be in pressure contact with the inner wall of the body lumen in the middle of the expansion. In this case, after the outer peripheral surface of the balloon member 40 is in pressure contact with the inner wall of the body lumen, the expansion rate in the width direction may become smaller, and the expansion rate in the axial direction may become larger.

As shown in Table 1, in the initial expansion stage of the balloon member 40, the ratio of the expansion rate in the axial direction of the balloon member 40 (also referred to as an axial expansion ratio) to the expansion rate in the radial direction of the balloon member 40 is equal to or more than 5.0%, preferably equal to or more than 7.5%, and more preferably equal to or more than 10.0%. When the dimension of the balloon member 40 in the axial direction is increased in advance in the initial expansion stage, and the balloon member 40 is arranged in the body lumen, the dimension of the balloon member 40 in the axial direction is in a large state before the outer peripheral surface of the balloon member 40 is in pressure contact with the inner wall of the body lumen. As a result, unnecessary pressure is prevented from being applied to the inner wall of the body lumen when the balloon member 40 is in pressure contact with the inner wall of the body lumen.

In the initial expansion stage, the axial expansion ratio is preferably less than 30.0%, more preferably less than 20.0%, and still more preferably less than 15.0%. By setting the ratio to be equal to or less than the above-described value, the balloon member 40 is sufficiently expanded in the width direction in the initial expansion stage. As a result, it is possible to achieve an object of sealing the body lumen at an earlier stage.

On the other hand, the axial expansion ratio in the late expansion stage is larger than the axial expansion ratio in the initial expansion stage. More specifically, the axial expansion ratio increases each time the expansion stage progresses. As described above, by configuring the balloon member 40 such that the amount of increase in the dimension of the balloon member 40 in the axial direction is larger as the balloon member 40 is expanded by the injection of the fluid, the expanded balloon member 40 is expanded in the axial direction, so that the balloon member 40 is less likely to be in pressure contact with the inner wall of the body lumen, and the pressure on the inner wall of the body lumen is relaxed.

In particular, as shown in Table 1, in the late expansion stage, the axial expansion ratio is equal to or more than 80.0%, preferably equal to or more than 100.0%, and more preferably equal to or more than 150.0%. By setting the ratio to be equal to or more than the above-described value and to a larger value in the late expansion stage than in the initial expansion stage, the balloon member 40 can be sufficiently expanded in the axial direction.

An upper limit of the ratio of the expansion rate in the axial direction to the expansion rate in the width direction in the late expansion stage is 250.0%, preferably 200.0%, and more preferably 175.0%. By restricting the expansion in the axial direction, the interference with the treatment tool or the like arranged in the vicinity of the balloon member 40 in the body lumen is suppressed.

As shown in FIG. 5, the attachment portion 13 is a portion of the tubular member 10 arranged on the inner diameter side (particularly, inside the hollow portion 42) of the balloon member 40. Specifically, the attachment portion 13 is a partial length region of the tube wall of the tubular member. The attachment portion 13 includes the spring forming portion 15 on the base end side and a spring non-forming portion 17 on the leading end side. The spring forming portion 15 is a portion in which a spring that is expandable and contractible in the axial direction is formed by forming the helical slit 44 or the like. The spring non-forming portion 17 is a portion in which the helical slit 44 is not formed and in which a spring that does not substantially expand or contract in the axial direction is not formed.

Since a portion of the attachment portion 13 on the leading end side does not form the spring, the portion does not have the flexibility of the spring forming portion 15 and is more rigid than the spring forming portion 15. Therefore, when the balloon member 40 is inflated and expanded in the axial direction, the balloon member 40 can be expanded straight with respect to the extending direction of the spring non-forming portion 17 that is a portion of the attachment portion 13 on the leading end side. In other words, when the entire attachment portion 13 is a spring when the balloon member 40 is expanded in the axial direction, the entire attachment portion 13 may have flexibility, and the balloon member 40 may be expanded while being unexpectedly bent or twisted. However, since the portion of the attachment portion 13 on the leading end side does not have large flexibility, the balloon member 40 can be expanded approximately in the axial direction.

In the present embodiment, a central portion of the attachment portion 13 in the axial direction is the spring forming portion 15. In other words, a length (length in the axial direction) of the spring forming portion 15 in the attachment portion 13 is larger than a length (length in the axial direction) of the spring non-forming portion 17 in the attachment portion 13.

Instead of the present embodiment, substantially the entire attachment portion 13 in the axial direction may be the spring forming portion 15. In addition, only the central portion of the attachment portion 13 in the axial direction may be the spring forming portion 15, and both end portions of the attachment portion 13 in the axial direction may be the spring non-forming portion 17.

The winding pitch in the second region 15a2 in the spring is smaller than the winding pitch in the first region 15a1 in the spring. In addition, the winding pitch in the third region 15a3 in the spring is larger than the winding pitch in the second region 15a2. Here, the winding pitch is a distance in the axial direction from one end on the leading end side of one turn to one end on the leading end side of another turn adjacent to the leading end side of the one turn. That is, the winding pitch can also be said to be a value that is the sum of the width dimension of the helical slit 44 and the width dimension of the ribbon 15a. In addition, in the present embodiment, the width of the helical slit 44 in the reduced-diameter state is substantially constant from the first region 15a1 to the third region 15a3. That is, the width of the ribbon 15a in the second region 15a2 in the spring is smaller than the width of the ribbon 15a in the first region 15a1 in the spring. Further, the width of the ribbon 15a in the third region 15a3 in the spring is larger than the width of the ribbon 15a in the second region 15a2.

The first region 15a1 is a predetermined length region on the base end side in the spring. The second region 15a2 is a length region on the leading end side with respect to the first region 15a1 in the spring. The third region 15a3 is a length region on the leading end side with respect to the second region 15a2 in the spring. In the present embodiment, the winding pitch in the second region 15a2 is the minimum value among the winding pitches of the attachment portion 13.

Since the winding pitch is small in the second region 15a2, the amount of the liquid flowing into the hollow portion 42 is increased, and the liquid can be favorably fed to the hollow portion 42. In addition, since the winding pitch in the third region 15a3 on the leading end side of the second region 15a2 is large, the tubular member 10 is prevented from being unexpectedly bent between the spring forming portion 15 and the spring non-forming portion 17.

In the present embodiment, the first region 15a1 to the third region 15a3 are all arranged in the attachment portion 13. The first region 15a1 to the third region 15a3 are particularly arranged inside the hollow portion 42. As described above, since the first region 15a1 and the third region 15a3, which do not have the minimum winding pitch, are arranged at both end portions of the attachment portion 13, the attachment portion 13 is prevented from having excessive flexibility.

In the large-diameter state or the small-diameter state, the width of the helical slit 44 in the second region 15a2 is preferably larger than the width of the helical slit 44 in the first region 15a1 or the third region 15a3. In this way, since the width of the helical slit 44 in the second region 15a2 is large, the hollow portion 42 and the lumen 11 can be sufficiently communicated with each other. In addition, since the width of the helical slit 44 in the first region 15a1 or the third region 15a3 is small, the first region 15a1 or the third region 15a3 is maintained to be rigid, and the attachment portion 13 can be prevented from having excessive flexibility. That is, with the above configuration, the hollow portion 42 and the lumen 11 can sufficiently communicate with each other in the second region 15a2 while the rigidity of the attachment portion 13 can be maintained within a desired range.

In the present embodiment, the spring is also formed in a portion (base end side forming portion 15d) of the tube wall on the base end side with respect to the attachment portion 13. That is, the base end side forming portion 15d is also a portion of the spring forming portion 15. More specifically, the spring is formed by a common helical slit 44 not only in the tube wall inside the hollow portion 42 but also in the tube wall arranged on the inner diameter side of the seal band 46 and the first adhesive 51 and the tube wall (tube wall in the base end side forming portion 15d) on the base end side with respect to the first adhesive 51. That is, the spring is integrally formed in the tube wall inside the hollow portion 42, the tube wall arranged on the inner diameter side of the seal band 46 and the first adhesive 51, and the tube wall in the base end side forming portion 15d.

The spring is also formed in the base end side forming portion 15d, and thus the base end side forming portion 15d has flexibility. As a result, when the balloon member 40 is arranged in the body lumen, the tubular member 10 in the vicinity of the balloon member 40 can follow the shape of the body lumen, and unnecessary load is prevented from being applied to the balloon member 40 and the member (for example, the adhesive 50) in the vicinity thereof. Therefore, the deformation of the balloon member 40 and the member in the vicinity thereof is suppressed, and, for example, the damage to the balloon member 40 or the liquid leakage from the balloon member 40 is suppressed.

The winding pitch of the spring formed in the base end side forming portion 15d is larger than the winding pitch in the third region 15a3. In other words, the width of the ribbon 15a in the base end side forming portion 15d is larger than the width of the ribbon 15a in the third region 15a3. As a result, the tubular member 10 is prevented from being easily bent between the spring forming portion 15 and the spring non-forming portion 17 on the base end side of the spring forming portion 15.

In the present embodiment, an angle of a portion of the ribbon 15a in the helical direction with respect to the axial direction in the reduced-diameter state is equal to or more than 60 degrees. More specifically, in the second region 15a2, the angle of the portion of the ribbon 15a in the helical direction with respect to the axial direction need only be equal to or more than 60 degrees. Instead of this, an angle of substantially the entire ribbon 15a in the attachment portion 13 in the helical direction with respect to the axial direction may be equal to or more than 60 degrees. Alternatively, the angle is more preferably equal to or more than 75 degrees. Therefore, the expansion and contraction of the spring forming portion 15 in the axial direction is more favorable. As a result, the balloon member 40 can be sufficiently expanded in the axial direction in the small-diameter state or the large-diameter state (expanded state).

In the small-diameter state or the large-diameter state, the angle of the portion of the ribbon 15a in the helical direction with respect to the axial direction may be equal to or more than 60 degrees in the same manner.

Next, a structure of the base end portion of the tubular member 10 will be described in detail.

As shown in FIG. 1 and (a) of FIG. 2, the first transition portion 25 is arranged between the leading end portion 26 and the intermediate portion 24. The second transition portion 23 is arranged between the intermediate portion 24 and the base end portion 22. The first transition portion 25 and the second transition portion 23 each have a tapered shape. The first transition portion 25 and the second transition portion 23, which are the transition portions, are partial length regions in the linear member 20, in which the rate of change in the outer diameter is larger than that of the base end portion 22, the intermediate portion 24, or the leading end portion 26 adjacent to the transition portion. The rate of change in the outer diameter is an amount of change in the outer diameter per a predetermined length. In the present embodiment, the outer diameters of the base end portion 22, the intermediate portion 24, and the leading end portion 26 are substantially uniform and do not change. In addition, the outer diameter of the linear member 20 gradually decreases from the base end portion 22 to the intermediate portion 24 or from the intermediate portion 24 to the leading end portion 26 in the second transition portion 23 or the first transition portion 25. Instead of the present embodiment, when the outer diameter of the base end portion 22, the intermediate portion 24, and the leading end portion 26 gradually increases or decreases toward the leading end at a small rate of change, a portion in which the outer diameter changes to be larger or smaller at a rate of change larger than the rate of change in the base end portion 22 or the like may be used as the transition portion.

The angle formed by the tapered shape in the second transition portion 23 with respect to the axial direction is larger than the angle formed by the tapered shape in the first transition portion 25 with respect to the axial direction. The rate of change in the outer diameter of the second transition portion 23 that decreases toward the leading end is larger than the rate of change in the outer diameter of the first transition portion 25 that decreases toward the leading end. The angle formed by the tapered shape in the second transition portion 23 or the first transition portion 25 with respect to the axial direction is an angle formed by the extending direction of the surface of the second transition portion 23 or the first transition portion 25 having the tapered shape and the axial direction. The extending direction (axial direction of the central axis of the first transition portion 25 or the second transition portion 23) of the first transition portion 25 or the second transition portion 23 may not be parallel to the axial direction of the tubular member 10 and may be in an intersecting or twisted relationship with each other due to the wave shape forming portion 24c described later. In this case, the angle between the tapered shape of the first transition portion 25 or the second transition portion 23 and the axial direction in a state in which the extending direction of the first transition portion 25 or the second transition portion 23 and the axial direction of the tubular member 10 are not parallel may be used as the angle formed by the tapered shape in the transition portion with respect to the axial direction. Alternatively, the angle between the tapered shape of the first transition portion 25 or the second transition portion 23 and the axial direction in a state in which the extending direction of the first transition portion 25 or the second transition portion 23 and the axial direction of the tubular member 10 are parallel may be used as the angle formed by the tapered shape in the transition portion with respect to the axial direction.

The outer diameter (wire diameter) of the base end portion 22 is larger than the width of the lumen 11. As shown in FIG. 1, when the linear member 20 is completely inserted into the tubular member 10, a portion of the second transition portion 23 on the leading end side is fitted into the lumen 11, and a portion of the second transition portion 23 on the base end side is arranged outside the lumen 11. In this case, a surface of the second transition portion 23 is in contact with a base end of the inner wall that defines the lumen 11.

With the above configuration, when the linear member 20 is inserted into the tubular member 10, the second transition portion 23 between the base end portion 22 and the intermediate portion 24 acts as a flange, and the linear member 20 is prevented from being excessively inserted into the tubular member 10 by the second transition portion 23. In addition, since the first transition portion 25 is a gentle taper, the linear member 20 is less likely to be bent in the first transition portion 25 where the wire diameter changes when the linear member 20 is pushed into the tubular member 10.

The length of the second transition portion 23 in the axial direction is preferably smaller than the length of the first transition portion 25 in the axial direction. This is because the second transition portion 23 is inclined more steeply with respect to the axial direction, and the first transition portion 25 can be inclined less steeply with respect to the axial direction.

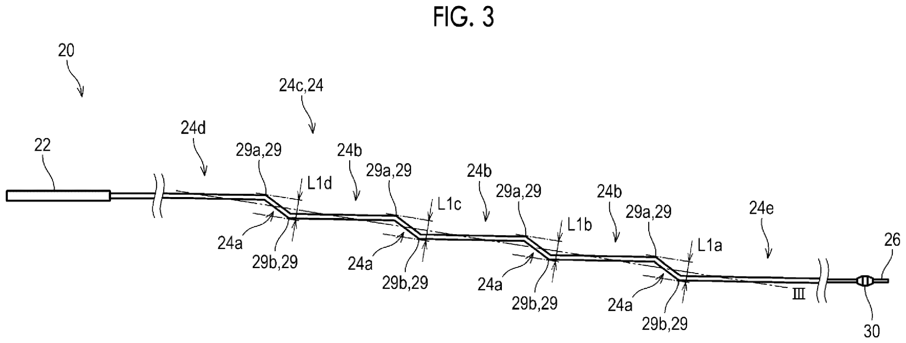

As shown in FIG. 3, the intermediate portion 24 of the linear member 20 has a wave shape. Specifically, the intermediate portion 24 is bent at a plurality of bent portions 29. In the present embodiment, the intermediate portion 24 is bent at eight bent portions 29. A plurality of first bent portions 29a and a plurality of second bent portions 29b, which are the bent portions 29, are alternately arranged in the intermediate portion 24. The intermediate portion 24 according to the present embodiment has four first bent portions 29a and four second bent portions 29b. The wave shape is formed by being bent upward or bent downward at each of the plurality of first bent portions 29a and the plurality of second bent portions 29b. The bent portion 29 is a length region (a curved or bent length region) in which the radius of curvature is smaller than that in the first length region 24a or the second length region 24b described later in the intermediate portion 24. Here, the upward bending is bending in one direction intersecting the axial direction, and the downward bending is bending in a direction (particularly, an opposite direction) different from the predetermined one direction of the upward bending. In the present embodiment, the linear member 20 is bent in a predetermined direction at the first bent portion 29a, and is bent in a direction opposite to the predetermined direction at the second bent portion 29b. That is, the linear member 20 is bent in the same direction at the plurality of first bent portions 29a, and the linear member 20 is bent in the same direction at the plurality of second bent portions 29b. Alternatively, the linear member 20 may be bent in different directions at each of the plurality of first bent portions 29a or each of the plurality of second bent portions 29b.

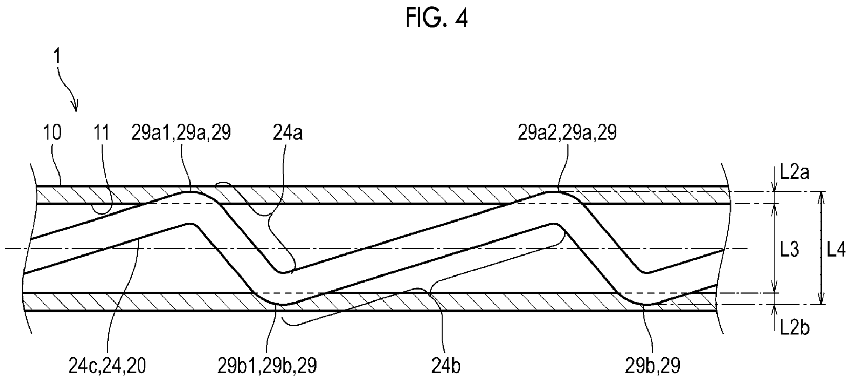

The right side in FIG. 4 is the leading end side, and the left side in FIG. 4 is the base end side. As will be described in detail below, in FIG. 4, the linear member 20 is shown in a shape when the linear member 20 is arranged outside the tubular member 10 (natural state). As shown in FIG. 4, the first length region 24a is a length region from one first bent portion 29a1 to a second bent portion 29b1 adjacent to one first bent portion 29a1 of the first bent portions 29a on the leading end side with respect to the one first bent portion 29a1. The second length region 24b is a length region from one second bent portion 29b1 of the second portions 29b to another first bent portion 29a2 of the first bent portions 29a adjacent to the one second bent portion 29b1 on the leading end side with respect to the one second bent portion 29b1. In the present embodiment, as shown in FIG. 3, in the intermediate portion 24, the first length region 24a and the second length region 24b are alternately arranged across the bent portion 29. A length of the first length region 24a from the one first bent portion 29a1 to the one second bent portion 29b1 is smaller than a length of the second length region 24b from the one second bent portion 29b1 to another first bent portion 29a2 adjacent to the one second bent portion 29b1 on the leading end side of the one second bent portion 29b1. That is, the length of the first length region 24a is smaller than the length of the second length region 24b adjacent to this first length region 24a on the leading end side. The length of the first length region 24a or the second length region 24b is a length of the first length region 24a or the second length region 24b in the extending direction of the first length region 24a or the second length region 24b.

In the present embodiment, the length of the first length region 24a is equal to or more than one-fourth and less than one-half of the length of the second length region 24b adjacent to that first length region on the leading end side.

The lengths of the plurality of first length regions 24a may be substantially the same as each other. The lengths of the plurality of second length regions 24b may be substantially the same as each other. The phrase "the lengths of the plurality of length regions are substantially the same as each other" means that each length takes a value of 90% to 110% of an average value of the plurality of length regions.

Alternatively, the length of one first length region 24a may be larger than the length of another first length region 24a located on the leading end side with respect to the one first length region 24a. Similarly, the length of one second length region 24b may be larger than the length of another second length region located on the leading end side with respect to the one second length region 24b. As a result, when the linear member 20 is pulled out, the linear member 20 is caught on the inner wall of the tubular member 10 at the bent portion 29 on the base end side of the intermediate portion 24, and when the linear member 20 is inserted, the sliding of the linear member 20 with respect to the tube wall at the bent portion 29 on the leading end side is favorable. This is because the amplitude in the intermediate portion 24 described later becomes smaller toward the leading end and becomes larger toward the base end. Specifically, when the bending angle at the bent portion 29 is constant, the amplitude of the linear member 20 in the vicinity of the first length region 24a or the second length region 24b is reduced due to the small length of the first length region 24a or the second length region 24b. When the amplitude is small, the force applied to the inner wall of the tubular member 10 by the linear member 20 is reduced. That is, when the amplitude is small, the sliding resistance of the linear member 20 with respect to the tubular member 10 is reduced.

Alternatively, the length of one first length region 24a may be smaller than the length of another first length region 24a located on the leading end side with respect to the one first length region 24a. Similarly, the length of one second length region 24b may be smaller than the length of another second length region located on the leading end side with respect to the one second length region 24b. As a result, the sliding resistance of a portion of the linear member 20 (intermediate portion 24) on the leading end side is increased, so that the movement of the sealing member 30 is prevented from being unexpected.

As shown in FIG. 4, the angle formed by the first length region 24a with respect to the axial direction is larger than the angle formed by the second length region 24b with respect to the axial direction.

With the above configuration, the first length region 24a is arranged to stand with respect to the axial direction. As a result, even when the linear member 20 is likely to be unexpectedly pulled out from the tubular member 10, the linear member 20 is prevented from being pulled out from the tubular member 10. This is because the linear member 20 is likely to be caught at the first bent portion 29a and is unlikely to be pulled out from the tubular member 10 even when the linear member 20 is to be pulled out from the tubular member 10.

In the intermediate portion 24, the amplitude of the wave shape in a portion on the leading end side is smaller than the amplitude of the wave shape in another portion on the base end side with respect to the portion on the leading end side.

As described above, since the amplitude in the leading end portion 26 is small, the linear member 20 is prevented from being caught on the tube wall of the tubular member at the leading end side of the wave shape forming portion 24c when the linear member 20 is inserted. Therefore, the linear member 20 can be favorably inserted into the tubular member 10.

The amplitude of the wave shape in the partial length region in the intermediate portion 24 is a maximum dimension of the partial length region in a direction orthogonal to the extending direction of the entire partial length region. The extending direction of the entire length region having the wave shape is the extending direction in which the wave shape is ignored. For example, the extending direction in the wave shape forming portion 24c is a direction indicated by a dashed line III in FIG. 3. In addition, the maximum dimension of the partial length region in the direction orthogonal to the extending direction of the entire partial length region is a maximum value of a distance between the first bent portion 29a and the second bent portion 29b along the direction orthogonal to the extending direction of the entire partial length region.

In the present embodiment, as shown in FIG. 3, an amplitude (for example, an amplitude L1a in FIG. 3) of the partial length region including the first bent portion 29a and the second bent portion 29b that are close to each other is smaller than an amplitude (for example, an amplitude L1b, an amplitude L1c, or an amplitude L1d in FIG. 3) of another partial length region including other first bent portions 29a and other second bent portions 29b that are close to each other at the base end side of the partial length region. The values of the amplitude L1a, the amplitude L1b, the amplitude L1c, and the amplitude L1d may be increased in this order.

In the present embodiment, the amplitude L1a in the partial length region including the first bent portion 29a and the second bent portion 29b that are arranged on the most leading end side in the intermediate portion 24 (partial length region including the first bent portion 29a and the second bent portion 29b that are arranged second from the leading end side) is smaller than the amplitude of the entire intermediate portion 24 (particularly, the wave shape forming portion 24c). In addition, the amplitude L1a is smaller than an average amplitude (average of the amplitude L1a to the amplitude L1d) of the length region in the vicinity of the group of the first bent portion 29a and the second bent portion 29b that are close to each other. Further, in the present embodiment, the amplitude L1a is smaller than any of the amplitudes L1b to L1d.

In the present embodiment, more specifically, in the intermediate portion 24, the amplitude of the wave shape in a portion on the leading end side may be substantially the same as the amplitude of the wave shape in another portion on the base end side with respect to the portion on the leading end side. That is, the amplitude of the wave shape in the intermediate portion 24 may be constant from the leading end to the base end. The amplitudes of the plurality of wave shape portions may be substantially the same, and the magnitude relationship of the amplitudes as described above may be established. The phrase "the amplitude of one portion and the amplitude of another portion are substantially the same" means that the amplitude of one portion is a value of equal to or more than 90% and equal to or less than 110% of the amplitude of the other portion adjacent to the one portion, and the maximum amplitude in the comparison target is equal to or less than 1.5 times the minimum amplitude in the comparison target. For example, when the amplitudes of two adjacent length regions are compared, the phrase "the amplitude of one portion and the amplitude of another portion are substantially the same" means that one amplitude is a value of equal to or more than 90% and equal to or less than 110% of the other amplitude. When the first bent portion 29a and the second bent portion 29b that are close to each other are grouped as in the present embodiment, there may be three or more groups, and there may be three or more amplitudes (amplitudes L1a to L1d). When it is determined whether the plurality of amplitudes are substantially the same as described above, the phrase "the amplitude of one portion and the amplitude of another portion are substantially the same" means that the amplitude of one portion is included in a range of 90% to 110% of the amplitude in another portion adjacent to the one portion, and the maximum value of the target amplitude is equal to or less than 1.5 times the minimum value of the target amplitude.

Alternatively, the amplitude of one portion and the amplitude of another portion need not be substantially the same as each other. That is, the amplitude of one portion may be a value of less than 90% of the amplitude of another portion, or may be a value of more than 110%. In addition, when the amplitudes of the plurality of length regions are compared, the maximum amplitude may be more than 1.5 times the minimum amplitude.

The amplitude of the wave shape forming portion 24c in a case of being arranged outside the tubular member 10 (in a natural state) is larger than the width (inner diameter of the tubular member 10) of the lumen 11 in the tubular member 10 as will be described later. That is, the amplitude L4 shown in FIG. 4 is larger than the inner diameter L3 of the tubular member 10. Therefore, the wave shape forming portion 24c is in pressure contact with the inner wall of the tubular member 10 at the bent portion 29. That is, the wave shape forming portion 24c arranged in the lumen 11 has a flattened shape as compared with the shape in the natural state. Specifically, the amplitude of the wave shape forming portion 24c in a case of being arranged in the lumen 11 is substantially the same as the width of the lumen 11, and the radius of curvature of the bent portion 29 is larger than the radius of curvature of the bent portion 29 in the natural state.

The radius of curvature of the bent portion 29 described in the upper part or described later may be the radius of curvature of the bent portion 29 in the wave shape forming portion 24c arranged in the lumen 11 or the radius of curvature of the bent portion 29 in the wave shape forming portion 24c in the natural state. In addition, the amplitude of the wave shape forming portion 24c described in the upper part or described later may be the amplitude of the wave shape forming portion 24c arranged in the lumen 11 or the amplitude of the wave shape forming portion 24c in the natural state.

For convenience of description, FIG. 4 shows the shape of the wave shape forming portion 24c in the natural state. Therefore, in FIG. 4, the bent portion 29 appears to be caught into the inner wall of the tubular member 10, but in reality, as described above, the bent portion 29 is deformed to be spread and is in pressure contact with the inner wall of the tubular member 10.

In the present embodiment, the radius of curvature of the bent portion 29 is smaller than the length of the second length region 24b.

As a result, by making the radius of curvature of the bent portion 29 smaller than the length of the second length region 24b, which is the longest straight portion in the wave shape forming portion 24c, to have a sharp shape, the linear member 20 is likely to be caught on the inner wall of the tubular member 10 at the bent portion 29. As a result, the linear member 20 is unlikely to be pulled out from the tubular member 10.

The bent portion 29 is bent into an arc shape of a perfect circle, and the radius of curvature may be constant over the entire length region of the bent portion 29. Alternatively, the radius of curvature need not be constant throughout the length region of the bent portion 29. In this case, the radius of curvature of the bent portion 29 may be an average radius of curvature of the bent portion 29, or may be a maximum value or a minimum value. Alternatively, the radius of curvature of the bent portion 29 may be a radius of curvature of a portion on the leading end side or a portion on the base end side of the bent portion 29.

The radius of curvature of the bent portion 29 is preferably larger than the length of the first length region 24a. As a result, when the linear member 20 is pulled out from the tubular member 10 or the like, even when the resistance between the linear member 20 and the inner wall of the tubular member 10 at the bent portion 29 (particularly, the first bent portion 29a) is large, the intermediate portion 24 is bent at the first bent portion 29a, and the linear member 20 is prevented from being pulled out by being caught at the first length region 24a.

In the present embodiment, the radius of curvature of one first bent portion 29a and the radius of curvature of one second bent portion 29b adjacent to the one first bent portion 29a across the first length region 24a are substantially the same. The phrase "the radius of curvature of the first bent portion 29a and the radius of curvature of the second bent portion 29b are substantially the same" means that the radius of curvature of the first bent portion 29a is equal to or more than two-thirds and equal to or less than 1.5 times the radius of curvature of the second bent portion 29b. It is preferable that the phrase "the radius of curvature of the first bent portion 29a and the radius of curvature of the second bent portion 29b are substantially the same" means that the radius of curvature of the first bent portion 29a is a value of equal to or more than 90% and equal to or less than 110% of the radius of curvature of the second bent portion 29b.

In addition, in the present embodiment, the radii of curvature of the first bent portions 29a or the radii of curvature of the second bent portions 29b are substantially the same as each other. The phrase "the radii of curvature of the first bent portions 29a or the radii of curvature of the second bent portions 29b are substantially the same as each other" means that one first bent portion 29a (or one second bent portion 29b) is a value of equal to or more than 90% and equal to or less than 110% of the radius of curvature of another first bent portion 29a (or another second bent portion 29b) adjacent to the one first bent portion 29a (or the one second bent portion 29b), and the maximum radius of curvature of the target first bent portion 29a (or the target second bent portion 29b) is 1.5 times the minimum radius of curvature of the target first bent portion 29a (or the target second bent portion 29b).

The present embodiment may further be configured such that the radius of curvature of one of the first bent portions 29a (one first bent portion 29a1) is smaller than the radius of curvature of one of the second bent portions 29b (one second bent portion 29b1) adjacent to the one first bent portion 29a1 across the first length region 24a. In other words, the radius of curvature of one first bent portion 29a1 of the first bent portions 29a is smaller than the radius of curvature of one second bent portion 29b1 of the second bent portions 29b adjacent to the one first bent portion 29a1 on the leading end side with respect to the one first bent portion 29a1.

As described above, by reducing the radius of curvature of the first bent portion 29a, the linear member 20 is likely to be caught at the first bent portion 29a in a case of pulling out the linear member 20, and the first length region is likely to stand with respect to the axial direction. As a result, the linear member 20 is prevented from being unexpectedly pulled out. On the other hand, since the first length region 24a and the second length region 24b across the second bent portion 29b1 are bent into a curved shape closer to a straight shape due to the large radius of curvature of the second bent portion 29b, the first length region 24a is unlikely to stand with respect to the axial direction in a case of inserting the linear member 20. As a result, in a case of inserting the linear member 20 into the tubular member 10, the first length region 24a is prevented from standing with respect to the axial direction, which makes it difficult to insert the linear member 20 into the tubular member 10.

The radius of curvature of one second bent portion 29b arranged on the leading end side may be larger than the radius of curvature of another second bent portion 29b arranged on the base end side with respect to the one second bent portion 29b. As a result, the intermediate portion 24 is likely to slide on the inner wall of the tubular member 10 toward the leading end side, and the sliding resistance with respect to the inner wall of the tubular member 10 is large toward the base end side. Therefore, the linear member 20 is unlikely to be unexpectedly pulled out from the tubular member 10, but the linear member 20 is likely to be inserted into the tubular member 10. Alternatively, the radius of curvature of one second bent portion 29b arranged on the leading end side may be smaller than the radius of curvature of another second bent portion 29b arranged on the base end side with respect to the one second bent portion 29b. As a result, the sliding resistance on the leading end side of the intermediate portion 24 can be increased. As a result, when an unexpected force is applied to the linear member 20, the movement of the sealing member 30 in the axial direction is prevented from being unexpected. In addition, instead of the present embodiment, the radii of curvature of the second bent portions 29b may be substantially the same as each other from the leading end to the base end.

Similarly, the radius of curvature of one first bent portion 29a arranged on the leading end side may be larger than the radius of curvature of another first bent portion 29a arranged on the base end side with respect to the one first bent portion 29a. Alternatively, instead of this, the radius of curvature of one first bent portion 29a arranged on the leading end side may be smaller than the radius of curvature of another first bent portion 29a arranged on the base end side with respect to the one first bent portion 29a. As a result, the sliding resistance of the intermediate portion 24 on the leading end side with respect to the inner wall of the tubular member 10 is sufficiently large. As a result, for example, when the sealing member 30 is arranged at the closed position for maintaining the balloon member 40 in the expanded-diameter state, the sealing member 30 is prevented from being shifted to the base end side due to the internal pressure of the lumen 11 or the like. In addition, instead of the present embodiment, the radii of curvature of the first bent portions 29a may be substantially the same as each other from the leading end to the base end.

As shown in FIG. 1 or 3, the intermediate portion 24 includes a wave shape forming portion 24c, a first wave shape non-forming portion (first non-forming portion 24d), and a second wave shape non-forming portion (second non-forming portion 24e). The wave shape forming portion 24c is a portion in which the wave shape is formed in the intermediate portion 24. The first non-forming portion 24d and the second non-forming portion 24e are wave shape non-forming portions in which the wave shape is not formed. The first non-forming portion 24d is a partial length region arranged on the base end side with respect to the wave shape forming portion 24c. The second non-forming portion 24e is a partial length region arranged on the leading end side with respect to the wave shape forming portion 24c.

As shown in FIG. 1, the length of the first non-forming portion 24d in the axial direction is smaller than the length of the second non-forming portion 24e in the axial direction.

As described above, since the wave shape forming portion 24c is arranged on the base end side of the linear member 20 (particularly, the intermediate portion 24), a force is likely to be applied to the wave shape forming portion 24c when a force is applied to the base end portion 22 for insertion and removal in the axial direction. As a result, the linear member 20 is prevented from being unexpectedly bent at the wave shape non-forming portion (particularly, the first non-forming portion 24d), and the wave shape forming portion 24c can slide more favorably on the inner wall of the tubular member 10.

The length of the first non-forming portion 24d in the axial direction is preferably smaller than the length of the wave shape forming portion 24c in the axial direction. As a result, when a force is applied to the base end portion 22 to insert the linear member 20 into the tubular member 10, the force is likely to be transmitted to the wave shape forming portion 24c. Further, the length of the second non-forming portion 24e in the axial direction is preferably larger than the length of the first length region 24a. As a result, the second non-forming portion 24e is arranged to sufficiently follow the axial direction. As a result, when the linear member 20 is inserted into the tubular member 10, the second non-forming portion 24e can slide the sealing member 30 straight in the axial direction.

In the present embodiment, a difference (hereinafter, also referred to as a clearance; the sum of the difference L2a and the difference L2b in FIG. 4) between an inner diameter (inner diameter L3 in FIG. 4) of the tubular member 10 and an amplitude (amplitude L4 in FIG. 4) of a portion (wave shape forming portion 24c) of the intermediate portion 24 having the wave shape when the linear member 20 is arranged outside the tubular member 10 (referred to as a natural state) is equal to or more than 0.095 mm and less than 0.135 mm. A force (hereinafter, referred to as a pull-out strength) required to relatively move the linear member 20 to the base end side of the tubular member 10 with respect to the tubular member 10 is equal to or more than 2.6 N and less than 6.5 N.

As described above, since the clearance is a sufficient value, the intermediate portion 24 is in pressure contact with the inner wall of the tubular member 10 at the bent portion 29, and the sliding resistance of the intermediate portion 24 with respect to the inner wall of the tubular member 10 is large. As a result, the linear member 20 is prevented from being unexpectedly pulled out from the tubular member 10. Further, since the pull-out strength is equal to or more than 2.6 N and less than 6.5 N, it is relatively easy to pull out the linear member 20 from the tubular member 10 using an instrument or the like, but it is difficult to pull out the linear member 20 from the tubular member 10 by gripping the base end portion 22 with a hand or the like, and the linear member 20 is prevented from being unexpectedly pulled out from the tubular member 10.

As described above, the inner diameter of the tubular member 10 is smaller than the amplitude of the wave shape forming portion 24c in the natural state. That is, the clearance is a value obtained by subtracting the inner diameter of the tubular member 10 from the amplitude of the wave shape forming portion 24c in the natural state.

The pull-out strength is a force required to fix the tubular member 10 and pull out the linear member 20 inserted into the tubular member 10 from the tubular member 10. The pulling out of the linear member 20 from the tubular member 10 is to move the linear member 20 relative to the tubular member 10 toward the base end side by a predetermined distance.

### (Catheter Set)

As shown in FIG. 1, the balloon-equipped guide wire 1 may be provided as a constituent member of a catheter set 100.

Specifically, the catheter set 100 includes the balloon-equipped guide wire 1 and a catheter 200. The balloon-equipped guide wire 1 is inserted into the catheter 200. Specifically, the catheter 200 includes a wire lumen 210 that extends along a longitudinal direction of the catheter 200, and the balloon-equipped guide wire 1 is inserted into the wire lumen 210. A width of the wire lumen 210 is larger than an outer diameter of the tubular member 10 (particularly, a portion on the base end side with respect to the balloon member 40). Specifically, after the balloon-equipped guide wire 1 is inserted into the body lumen, the catheter 200 can advance toward the leading end along the outer layer side of the balloon-equipped guide wire 1 while the base end portion of the balloon-equipped guide wire 1 is passed through the wire lumen 210. In the present embodiment, the width of the wire lumen 210 may be smaller than or larger than the width of the balloon member 40 in the reduced-diameter state or the expanded state. As a result, the catheter 200 that has advanced in the leading end direction in the body lumen along the balloon-equipped guide wire 1 can stop at a position on the base end side with respect to the balloon member 40. In the present embodiment, the wire lumen 210 is provided only in a portion of the catheter 200 on the leading end side.

The catheter 200 includes a main lumen 220 that extends in the longitudinal direction of the catheter 200 and is different from the wire lumen 210. The catheter 200 supplies a drug to the body lumen through the main lumen 220 or suctions and removes a substance or the like in the body lumen.

It is preferable that an opening end portion (leading end portion) of the wire lumen 210 in the catheter 200 is arranged to protrude to the leading end side (distal side) with respect to the opening of the main lumen 220. In other words, a portion of the catheter 200 that forms the opening of the wire lumen 210 protrudes to the leading end side with respect to a portion of the catheter 200 that forms the opening of the main lumen 220. A leading end portion (particularly, a portion in which the main lumen 220 can be formed) of the catheter 200 according to the present embodiment has a shape that is cut out in a smooth curved surface. As a result, the leading end of the wire lumen 210 is arranged on a more leading end side of the catheter 200 than the leading end of the main lumen 220. Alternatively, the leading end of the wire lumen 210 may be arranged at the most leading end of the catheter 200 by forming the wire lumen 210 on the leading end side with respect to the opening of the catheter 200 such that the opening end of the wire lumen 210 is arranged on the leading end side with respect to the opening of the main lumen 220.

### <Second Embodiment>

FIG. 1 is a schematic plan view showing an example of a medical instrument 1 (balloon-equipped guide wire 1) according to the present embodiment.

A medical instrument according to the second embodiment includes: a tubular member; and a linear member that is arranged in a lumen at a base end portion of the tubular member and is movable in an axial direction of the tubular member, in which a sealing member that is in close contact with a portion of an inner wall that defines the lumen of the tubular member is provided at a leading end portion of the linear member, the linear member includes the leading end portion, an intermediate portion on a base end side with respect to the leading end portion, and a base end portion on the base end side with respect to the intermediate portion, the leading end portion, the intermediate portion, and the base end portion each have a partial length region having a uniform outer diameter, the outer diameter of the partial length region of the intermediate portion is smaller than 85% of a width of the lumen of the tubular member, and among the partial length regions of the leading end portion, the intermediate portion, and the base end portion, the partial length region of the base end portion is the thickest, and the partial length region of the leading end portion is the thinnest.

### (Medical Instrument)

First, an overview of the medical instrument 1 according to the present embodiment will be described.

The medical instrument 1 includes a tubular member 10 and a linear member 20. The linear member 20 is arranged in the lumen 11 at a base end portion 22 of the tubular member 10. The linear member 20 is movable in an axial direction of the tubular member 10. A sealing member 30 is provided around the leading end portion 26 of the linear member 20. The sealing member 30 is in close contact with a portion of the inner wall of the tubular member 10.

The linear member 20 includes a leading end portion 26, an intermediate portion 24, and a base end portion 22. The intermediate portion 24 is a portion of the linear member 20 on the base end side with respect to the leading end portion 26. The base end portion 22 is a portion of the linear member 20 on the base end side with respect to the intermediate portion 24. The leading end portion 26, the intermediate portion 24, and the base end portion 22 each have a partial length region having a uniform outer diameter.

An outer diameter of the partial length region having a uniform diameter in the intermediate portion 24 is smaller than 85% of the width of the lumen 11 of the tubular member 10. Among the partial length regions of the leading end portion 26, the intermediate portion 24, and the base end portion 22, the partial length region of the base end portion 22 is the thickest, and the partial length region of the leading end portion 26 is the thinnest.

With the above configuration, since the leading end portion of the linear member is thin and has flexibility, the leading end portion of the linear member can be deformed, such as bending, when the linear member is unexpectedly pushed into the tubular member. Therefore, the sealing member is prevented from immediately moving when the linear member is pushed into the tubular member. As a result, it is possible to provide a medical instrument in which the movement of the sealing member in the lumen is favorably controlled.

Specifically, since the leading end portion 26 of the linear member 20 is thin and has flexibility, the movement of the sealing member 30 in the lumen 11 is favorably controlled. For example, when the linear member 20 is unexpectedly pushed into the tubular member 10, the leading end portion 26 of the linear member 20 is bent. Therefore, the sealing member 30 is prevented from immediately moving when the linear member 20 is pushed into the tubular member 10. For example, even when the linear member 20 is unexpectedly inserted into the lumen 11 (into the leading end side) when the sealing member 30 is arranged on the proximal side of the injection port 18 (arranged at the open position described later) and the liquid or the like is injected from the injection port 18 into the lumen 11, the movement of the sealing member 30 to immediately close a part or all of the injection port 18 is suppressed. Therefore, it is easy to accurately inject a predetermined amount of the liquid into the lumen 11 without hindering the injection of the predetermined amount of the liquid even when the injection port 18 is unexpectedly closed.

In addition, as will be described later, the sealing member 30 according to the present embodiment is arranged on the outer diameter side of the leading end portion 26 of the linear member 20.

Since the leading end portion arranged on the inner diameter side of the sealing member 30 has a small diameter, the sealing member 30 can be formed to be sufficiently thick (large diameter) to be favorably in pressure contact with the inner wall of the tubular member 10, so that the sealing property of the lumen 11 by the sealing member 30 can be improved. Specifically, by making the leading end portion 26 of the linear member 20 thin, it is possible to increase a distance from a surface of the leading end portion 26 to the inner wall (inner wall forming the lumen 11) of the tubular member 10. In order to favorably seal the lumen 11 by sufficiently pressing the sealing member 30 against the inner wall of the tubular member 10 to increase the sliding resistance of the sealing member 30 on the inner wall, the sealing member 30 may be formed to be thick beyond the distance from the surface of the leading end portion 26 to the inner wall of the tubular member 10. In general, when the sealing member 30 is formed to be thick beyond the distance from the surface of the leading end portion 26 to the inner wall of the tubular member 10, a larger force is required to insert the sealing member 30 into the tubular member 10, and it may be difficult to insert the sealing member 30 into the lumen 11. However, when the leading end portion 26 of the linear member 20 is thin and the distance from the surface of the leading end portion 26 to the inner wall of the tubular member 10 is large, the sealing member 30 is sufficiently thick and thus can be deformed by having elasticity. Therefore, the sealing member 30 can be easily inserted into the tubular member 10.

In addition, since the wire diameter increases in the order of the leading end portion 26, the intermediate portion 24, and the base end portion 22, a force (a force applied to the base end portion 22 from the base end side toward the leading end side) applied to the base end portion 22 in the axial direction is likely to be transmitted to the leading end portion 26, and the sealing member 30 formed at the leading end portion 26 can be more favorably slid in the lumen 11.

The medical instrument 1 according to the present embodiment is characterized in that, regardless of an aspect of the expansion of the balloon member 40 in the balloon-equipped guide wire 1 (medical instrument 1) described in the first embodiment, the diameters of the plurality of partial length regions in the linear member 20 have a predetermined size relationship.

Next, the medical instrument 1 according to the present embodiment will be described in detail. Descriptions that overlap with the description of the medical instrument 1 according to the first embodiment will be omitted as appropriate.

The medical instrument 1 (balloon-equipped guide wire 1) according to the present embodiment is an instrument for plugging an opening provided in a hollow portion or a tubular organ in the body lumen in the living body. An instrument for hemostasis that is inserted into the blood vessel or the like is an example of the medical instrument 1. Further, the medical instrument 1 according to the present embodiment is a member that is inserted into a particularly elongated body lumen and is an elongated member as a whole. A guide wire or a catheter that is inserted into the blood vessel or the like is an example of the medical instrument 1. The medical instrument 1 according to the present embodiment is a balloon-equipped guide wire. More specifically, as shown in FIG. 1, the medical instrument 1 according to the present embodiment is a balloon-equipped guide wire 1 having a balloon on a portion on the leading end side. That is, the tubular member 10 has a balloon member 40 on the outer diameter side of a portion on the leading end side.

As shown in FIG. 1 and (a) of FIG. 2, the first transition portion 25 is arranged between the leading end portion 26 and the intermediate portion 24. The second transition portion 23 is arranged between the intermediate portion 24 and the base end portion 22. The first transition portion 25 and the second transition portion 23 each have a tapered shape. The first transition portion 25 and the second transition portion 23, which are the transition portions, are partial length regions in the linear member 20, in which the rate of change in the outer diameter is larger than that of the base end portion 22, the intermediate portion 24, or the leading end portion 26 adjacent to the transition portion. The rate of change in the outer diameter is an amount of change in the outer diameter per a predetermined length. In the present embodiment, the outer diameters of the base end portion 22, the intermediate portion 24, and the leading end portion 26 are substantially uniform and do not change. In addition, the outer diameter of the linear member 20 gradually decreases from the base end portion 22 to the intermediate portion 24 or from the intermediate portion 24 to the leading end portion 26 in the second transition portion 23 or the first transition portion 25. Instead of the present embodiment, when the outer diameter of the base end portion 22, the intermediate portion 24, and the leading end portion 26 gradually increases or decreases toward the leading end at a small rate of change, a portion in which the outer diameter changes to be larger or smaller at a rate of change larger than the rate of change in the base end portion 22 or the like may be used as the transition portion.

The angle formed by the tapered shape in the second transition portion 23 with respect to the axial direction is larger than the angle formed by the tapered shape in the first transition portion 25 with respect to the axial direction. The rate of change in the outer diameter of the second transition portion 23 that decreases toward the leading end is larger than the rate of change in the outer diameter of the first transition portion 25 that decreases toward the leading end. The angle formed by the tapered shape in the first transition portion 25 or the second transition portion 23 with respect to the axial direction is an angle formed by an extending direction of a surface of the first transition portion 25 or the second transition portion 23 having the tapered shape and the axial direction. The extending direction (axial direction of the central axis of the first transition portion 25 or the second transition portion 23) of the first transition portion 25 or the second transition portion 23 may not be parallel to the axial direction of the tubular member 10 and may be in an intersecting or twisted relationship with each other due to the wave shape forming portion 24c described later. In this case, the angle between the tapered shape of the first transition portion 25 or the second transition portion 23 and the axial direction in a state in which the extending direction of the first transition portion 25 or the second transition portion 23 and the axial direction of the tubular member 10 are not parallel may be used as the angle formed by the tapered shape in the transition portion with respect to the axial direction. Alternatively, the angle between the tapered shape of the first transition portion 25 or the second transition portion 23 and the axial direction in a state in which the extending direction of the first transition portion 25 or the second transition portion 23 and the axial direction of the tubular member 10 are parallel may be used as the angle formed by the tapered shape in the transition portion with respect to the axial direction.

The outer diameter (wire diameter) of the base end portion 22 is larger than the width of the lumen 11. As shown in FIG. 1, when the linear member 20 is completely inserted into the tubular member 10, a portion of the second transition portion 23 on the leading end side is fitted into the lumen 11, and a portion of the second transition portion 23 on the base end side is arranged outside the lumen 11. In this case, a surface of the second transition portion 23 is in contact with a base end of the inner wall that defines the lumen 11.

With the above configuration, when the linear member 20 is inserted into the tubular member 10, the second transition portion 23 between the base end portion 22 and the intermediate portion 24 acts as a flange, and the linear member 20 is prevented from being excessively inserted into the tubular member 10 by the second transition portion 23. In addition, since the first transition portion 25 is a gentle taper, the linear member 20 is less likely to be bent in the first transition portion 25 where the wire diameter changes when the linear member 20 is pushed into the tubular member 10.

The length of the second transition portion 23 in the axial direction is preferably smaller than the length of the first transition portion 25 in the axial direction. This is because the second transition portion 23 is inclined more steeply with respect to the axial direction, and the first transition portion 25 can be inclined less steeply with respect to the axial direction.

As shown in FIG. 3, the intermediate portion 24 of the linear member 20 has a wave shape. Specifically, the intermediate portion 24 is bent at a plurality of bent portions 29. In the present embodiment, the intermediate portion 24 is bent at eight bent portions 29. A plurality of first bent portions 29a and a plurality of second bent portions 29b, which are the bent portions 29, are alternately arranged in the intermediate portion 24. The intermediate portion 24 according to the present embodiment has four first bent portions 29a and four second bent portions 29b. The wave shape is formed by being bent upward or bent downward at each of the plurality of first bent portions 29a and the plurality of second bent portions 29b. The bent portion 29 is a length region (a curved or bent length region) in which the radius of curvature is smaller than that in the first length region 24a or the second length region 24b described later in the intermediate portion 24. Here, the upward bending is bending in one direction intersecting the axial direction, and the downward bending is bending in a direction (particularly, an opposite direction) different from the predetermined one direction of the upward bending. In the present embodiment, the linear member 20 is bent in a predetermined direction at the first bent portion 29a, and is bent in a direction opposite to the predetermined direction at the second bent portion 29b. That is, the linear member 20 is bent in the same direction at the plurality of first bent portions 29a, and the linear member 20 is bent in the same direction at the plurality of second bent portions 29b. Alternatively, the linear member 20 may be bent in different directions at each of the plurality of first bent portions 29a or each of the plurality of second bent portions 29b.

The right side in FIG. 4 is the leading end side, and the left side in FIG. 4 is the base end side. As will be described in detail below, in FIG. 4, the linear member 20 is shown in a shape when the linear member 20 is arranged outside the tubular member 10 (natural state). As shown in FIG. 4, the first length region 24a is a length region from one first bent portion 29a1 to a second bent portion 29b1 adjacent to one first bent portion 29a1 of the first bent portions 29a on the leading end side with respect to the one first bent portion 29a1. The second length region 24b is a length region from one second bent portion 29b1 of the second bent portion 29b to another first bent portion 29a2 of the first bent portions 29a adjacent to the one second bent portion 29b1 on the leading end side with respect to the one second bent portion 29b1. In the present embodiment, as shown in FIG. 3, in the intermediate portion 24, the first length region 24a and the second length region 24b are alternately arranged across the bent portion 29. A length of the first length region 24a from the one first bent portion 29a1 to the one second bent portion 29b1 is smaller than a length of the second length region 24b from the one second bent portion 29b1 to another first bent portion 29a2 adjacent to the one second bent portion 29b1 on the leading end side with respect to the one second bent portion 29b1. That is, the length of the first length region 24a is smaller than the length of the second length region 24b adjacent to this first length region 24a on the leading end side. The length of the first length region 24a or the second length region 24b is a length of the first length region 24a or the second length region 24b in the extending direction of the first length region 24a or the second length region 24b.

In the present embodiment, the length of the first length region 24a is equal to or more than one-fourth and less than one-half of the length of the second length region 24b adjacent to that first length region on the leading end side.

The lengths of the plurality of first length regions 24a may be substantially the same as each other. The lengths of the plurality of second length regions 24b may be substantially the same as each other. The phrase "the lengths of the plurality of length regions are substantially the same as each other" means that each length takes a value of 90% to 110% of an average value of the plurality of length regions.

Alternatively, the length of one first length region 24a may be larger than the length of another first length region 24a located on the leading end side with respect to the one first length region 24a. Similarly, the length of one second length region 24b may be larger than the length of another second length region located on the leading end side with respect to the one second length region 24b. As a result, when the linear member 20 is pulled out, the linear member 20 is caught on the inner wall of the tubular member 10 at the bent portion 29 on the base end side of the intermediate portion 24, and when the linear member 20 is inserted, the sliding of the linear member 20 with respect to the tube wall at the bent portion 29 on the leading end side is favorable. This is because the amplitude in the intermediate portion 24 described later becomes smaller toward the leading end and becomes larger toward the base end. Specifically, when the bending angle at the bent portion 29 is constant, the amplitude of the linear member 20 in the vicinity of the first length region 24a or the second length region 24b is reduced due to the small length of the first length region 24a or the second length region 24b. When the amplitude is small, the force applied to the inner wall of the tubular member 10 by the linear member 20 is reduced. That is, when the amplitude is small, the sliding resistance of the linear member 20 with respect to the tubular member 10 is reduced.

Alternatively, the length of one first length region 24a may be smaller than the length of another first length region 24a located on the leading end side with respect to the one first length region 24a. Similarly, the length of one second length region 24b may be smaller than the length of another second length region located on the leading end side with respect to the one second length region 24b. As a result, the sliding resistance of a portion of the linear member 20 (intermediate portion 24) on the leading end side is increased, so that the movement of the sealing member 30 is prevented from being unexpected.

As shown in FIG. 4, the angle formed by the first length region 24a with respect to the axial direction is larger than the angle formed by the second length region 24b with respect to the axial direction.

With the above configuration, the first length region 24a is arranged to stand with respect to the axial direction. As a result, even when the linear member 20 is likely to be unexpectedly pulled out from the tubular member 10, the linear member 20 is prevented from being pulled out from the tubular member 10. This is because the linear member 20 is likely to be caught at the first bent portion 29a and is unlikely to be pulled out from the tubular member 10 even when the linear member 20 is to be pulled out from the tubular member 10.

In the intermediate portion 24, the amplitude of the wave shape in a portion on the leading end side is smaller than the amplitude of the wave shape in another portion on the base end side with respect to the portion on the leading end side.

As described above, since the amplitude in the leading end portion 26 is small, the linear member 20 is prevented from being caught on the tube wall of the tubular member 10 at the leading end side of the wave shape forming portion 24c when the linear member 20 is inserted. Therefore, the linear member 20 can be favorably inserted into the tubular member 10.

The amplitude of the wave shape in the partial length region in the intermediate portion 24 is a maximum dimension of the partial length region in a direction orthogonal to the extending direction of the entire partial length region. The extending direction of the entire length region having the wave shape is the extending direction in which the wave shape is ignored. For example, the extending direction in the wave shape forming portion 24c is a direction indicated by a dashed line III in FIG. 3. In addition, the maximum dimension of the partial length region in the direction orthogonal to the extending direction of the entire partial length region is a maximum value of a distance between the first bent portion 29a and the second bent portion 29b along the direction orthogonal to the extending direction of the entire partial length region.

In the present embodiment, as shown in FIG. 3, an amplitude (for example, an amplitude L1a in FIG. 3) of the partial length region including the first bent portion 29a and the second bent portion 29b that are close to each other is smaller than an amplitude (for example, an amplitude L1b, an amplitude L1c, or an amplitude L1d in FIG. 3) of another partial length region including other first bent portions 29a and other second bent portions 29b that are close to each other at the base end side of the partial length region. The values of the amplitude L1a, the amplitude L1b, the amplitude L1c, and the amplitude L1d may be increased in this order.

In the present embodiment, the amplitude L1a in the partial length region including the first bent portion 29a and the second bent portion 29b that are arranged on the most leading end side in the intermediate portion 24 (partial length region including the first bent portion 29a and the second bent portion 29b that are arranged second from the leading end side) is smaller than the amplitude of the entire intermediate portion 24 (particularly, the wave shape forming portion 24c). In addition, the amplitude L1a is smaller than an average amplitude (average of the amplitude L1a to the amplitude L1d) of the length region in the vicinity of the group of the first bent portion 29a and the second bent portion 29b that are close to each other. Further, in the present embodiment, the amplitude L1a is smaller than any of the amplitudes L1b to L1d.

In the present embodiment, more specifically, in the intermediate portion 24, the amplitude of the wave shape in a portion on the leading end side may be substantially the same as the amplitude of the wave shape in another portion on the base end side with respect to the portion on the leading end side. That is, the amplitude of the wave shape in the intermediate portion 24 may be constant from the leading end to the base end. The amplitudes of the plurality of wave shape portions may be substantially the same, and the magnitude relationship of the amplitudes as described above may be established. The phrase "the amplitude of one portion and the amplitude of another portion are substantially the same" means that the amplitude of one portion is a value of equal to or more than 90% and equal to or less than 110% of the amplitude of the other portion adjacent to the one portion, and the maximum amplitude in the comparison target is equal to or less than 1.5 times the minimum amplitude in the comparison target. For example, when the amplitudes of two adjacent length regions are compared, the phrase "the amplitude of one portion and the amplitude of another portion are substantially the same" means that one amplitude is a value of equal to or more than 90% and equal to or less than 110% of the other amplitude. When the first bent portion 29a and the second bent portion 29b that are close to each other are grouped as in the present embodiment, there may be three or more groups, and there may be three or more amplitudes (amplitudes L1a to L1d). When it is determined whether the plurality of amplitudes are substantially the same as described above, the phrase "the amplitude of one portion and the amplitude of another portion are substantially the same" means that the amplitude of one portion is included in a range of 90% to 110% of the amplitude in another portion adjacent to the one portion, and the maximum value of the target amplitude is equal to or less than 1.5 times the minimum value of the target amplitude.

Alternatively, the amplitude of one portion and the amplitude of another portion need not be substantially the same as each other. That is, the amplitude of one portion may be a value of less than 90% of the amplitude of another portion, or may be a value of more than 110%. In addition, when the amplitudes of the plurality of length regions are compared, the maximum amplitude may be more than 1.5 times the minimum amplitude.

The amplitude of the wave shape forming portion 24c in a case of being arranged outside the tubular member 10 (in a natural state) is larger than the width (inner diameter of the tubular member 10) of the lumen 11 in the tubular member 10 as will be described later. That is, the amplitude L4 shown in FIG. 4 is larger than the inner diameter L3 of the tubular member 10. Therefore, the wave shape forming portion 24c is in pressure contact with the inner wall of the tubular member 10 at the bent portion 29. That is, the wave shape forming portion 24c arranged in the lumen 11 has a flattened shape as compared with the shape in the natural state. Specifically, the amplitude of the wave shape forming portion 24c in a case of being arranged in the lumen 11 is substantially the same as the width of the lumen 11, and the radius of curvature of the bent portion 29 is larger than the radius of curvature of the bent portion 29 in the natural state.

The radius of curvature of the bent portion 29 described in the upper part or described later may be the radius of curvature of the bent portion 29 in the wave shape forming portion 24c arranged in the lumen 11 or the radius of curvature of the bent portion 29 in the wave shape forming portion 24c in the natural state. In addition, the amplitude of the wave shape forming portion 24c described in the upper part or described later may be the amplitude of the wave shape forming portion 24c arranged in the lumen 11 or the amplitude of the wave shape forming portion 24c in the natural state.

For convenience of description, FIG. 4 shows the shape of the wave shape forming portion 24c in the natural state. Therefore, in FIG. 4, the bent portion 29 appears to be caught into the inner wall of the tubular member 10, but in reality, as described above, the bent portion 29 is deformed to be spread and is in pressure contact with the inner wall of the tubular member 10.

In the present embodiment, the radius of curvature of the bent portion 29 is smaller than the length of the second length region 24b.

As a result, by making the radius of curvature of the bent portion 29 smaller than the length of the second length region 24b, which is the longest straight portion in the wave shape forming portion 24c, to have a sharp shape, the linear member 20 is likely to be caught on the inner wall of the tubular member 10 at the bent portion 29. As a result, the linear member 20 is unlikely to be pulled out from the tubular member 10.

The bent portion 29 is bent into an arc shape of a perfect circle, and the radius of curvature may be constant over the entire length region of the bent portion 29. Alternatively, the radius of curvature need not be constant throughout the length region of the bent portion 29. In this case, the radius of curvature of the bent portion 29 may be an average radius of curvature of the bent portion 29, or may be a maximum value or a minimum value. Alternatively, the radius of curvature of the bent portion 29 may be a radius of curvature of a portion on the leading end side or a portion on the base end side of the bent portion 29.

The radius of curvature of the bent portion 29 is preferably larger than the length of the first length region 24a. As a result, when the linear member 20 is pulled out from the tubular member 10 or the like, even when the resistance between the linear member 20 and the inner wall of the tubular member 10 at the bent portion 29 (particularly, the first bent portion 29a) is large, the intermediate portion 24 is bent at the first bent portion 29a, and the linear member 20 is prevented from being pulled out by being caught at the first length region 24a.

In the present embodiment, the radius of curvature of one first bent portion 29a and the radius of curvature of one second bent portion 29b adjacent to the one first bent portion 29a across the first length region 24a are substantially the same. The phrase "the radius of curvature of the first bent portion 29a and the radius of curvature of the second bent portion 29b are substantially the same" means that the radius of curvature of the first bent portion 29a is equal to or more than two-thirds and equal to or less than 1.5 times the radius of curvature of the second bent portion 29b. It is preferable that the phrase "the radius of curvature of the first bent portion 29a and the radius of curvature of the second bent portion 29b are substantially the same" means that the radius of curvature of the first bent portion 29a is a value of equal to or more than 90% and equal to or less than 110% of the radius of curvature of the second bent portion 29b.

In addition, in the present embodiment, the radii of curvature of the first bent portions 29a or the radii of curvature of the second bent portions 29b are substantially the same as each other. The phrase "the radii of curvature of the first bent portions 29a or the radii of curvature of the second bent portions 29b are substantially the same as each other" means that one first bent portion 29a (or one second bent portion 29b) is a value of equal to or more than 90% and equal to or less than 110% of the radius of curvature of another first bent portion 29a (or another second bent portion 29b) adjacent to the one first bent portion 29a (or the one second bent portion 29b), and the maximum radius of curvature of the target first bent portion 29a (or the target second bent portion 29b) is 1.5 times the minimum radius of curvature of the target first bent portion 29a (or the target second bent portion 29b).

The present embodiment may further be configured such that the radius of curvature of one of the first bent portions 29a (one first bent portion 29a1) is smaller than the radius of curvature of one of the second bent portions 29b (one second bent portion 29b1) adjacent to the one first bent portion 29a1 across the first length region 24a. In other words, the radius of curvature of one first bent portion 29a1 of the first bent portions 29a is smaller than the radius of curvature of one second bent portion 29b1 of the second bent portions 29b adjacent to the one first bent portion 29a1 on the leading end side with respect to the one first bent portion 29a1.

As described above, by reducing the radius of curvature of the first bent portion 29a, the linear member 20 is likely to be caught at the first bent portion 29a in a case of pulling out the linear member 20, and the first length region is likely to stand with respect to the axial direction. As a result, the linear member 20 is prevented from being unexpectedly pulled out. On the other hand, since the first length region 24a and the second length region 24b across the second bent portion 29b1 are bent into a curved shape closer to a straight shape due to the large radius of curvature of the second bent portion 29b, the first length region 24a is unlikely to stand with respect to the axial direction in a case of inserting the linear member 20. As a result, in a case of inserting the linear member 20 into the tubular member 10, the first length region 24a is prevented from standing with respect to the axial direction, which makes it difficult to insert the linear member 20 into the tubular member 10.

The radius of curvature of one second bent portion 29b arranged on the leading end side may be larger than the radius of curvature of another second bent portion 29b arranged on the base end side with respect to the one second bent portion 29b. As a result, the intermediate portion 24 is likely to slide on the inner wall of the tubular member 10 toward the leading end side, and the sliding resistance with respect to the inner wall of the tubular member 10 is large toward the base end side. Therefore, the linear member 20 is unlikely to be unexpectedly pulled out from the tubular member 10, but the linear member 20 is likely to be inserted into the tubular member 10. Alternatively, the radius of curvature of one second bent portion 29b arranged on the leading end side may be smaller than the radius of curvature of another second bent portion 29b arranged on the base end side with respect to the one second bent portion 29b. As a result, the sliding resistance on the leading end side of the intermediate portion 24 can be increased. As a result, when an unexpected force is applied to the linear member 20, the movement of the sealing member 30 in the axial direction is prevented from being unexpected. In addition, instead of the present embodiment, the radii of curvature of the second bent portions 29b may be substantially the same as each other from the leading end to the base end.

Similarly, the radius of curvature of one first bent portion 29a arranged on the leading end side may be larger than the radius of curvature of another first bent portion 29a arranged on the base end side with respect to the one first bent portion 29a. Alternatively, instead of this, the radius of curvature of one first bent portion 29a arranged on the leading end side may be smaller than the radius of curvature of another first bent portion 29a arranged on the base end side with respect to the one first bent portion 29a. As a result, the sliding resistance of the intermediate portion 24 on the leading end side with respect to the inner wall of the tubular member 10 is sufficiently large. As a result, for example, when the sealing member 30 is arranged at the closed position for maintaining the balloon member 40 in the expanded-diameter state, the sealing member 30 is prevented from being shifted to the base end side due to the internal pressure of the lumen 11 or the like. In addition, instead of the present embodiment, the radii of curvature of the first bent portions 29a may be substantially the same as each other from the leading end to the base end.

As shown in FIG. 1 or 3, the intermediate portion 24 includes a wave shape forming portion 24c, a first wave shape non-forming portion (first non-forming portion 24d), and a second wave shape non-forming portion (second non-forming portion 24e). The wave shape forming portion 24c is a portion in which the wave shape is formed in the intermediate portion 24. The first non-forming portion 24d and the second non-forming portion 24e are wave shape non-forming portions in which the wave shape is not formed. The first non-forming portion 24d is a partial length region arranged on the base end side with respect to the wave shape forming portion 24c. The second non-forming portion 24e is a partial length region arranged on the leading end side with respect to the wave shape forming portion 24c.

As shown in FIG. 1, the length of the first non-forming portion 24d in the axial direction is smaller than the length of the second non-forming portion 24e in the axial direction.

As described above, since the wave shape forming portion 24c is arranged on the base end side of the linear member 20 (particularly, the intermediate portion 24), a force is likely to be applied to the wave shape forming portion 24c when a force is applied to the base end portion 22 for insertion and removal in the axial direction. As a result, the linear member 20 is prevented from being unexpectedly bent at the wave shape non-forming portion (particularly, the first non-forming portion 24d), and the wave shape forming portion 24c can slide more favorably on the inner wall of the tubular member 10.

The length of the first non-forming portion 24d in the axial direction is preferably smaller than the length of the wave shape forming portion 24c in the axial direction. As a result, when a force is applied to the base end portion 22 to insert the linear member 20 into the tubular member 10, the force is likely to be transmitted to the wave shape forming portion 24c. Further, the length of the second non-forming portion 24e in the axial direction is preferably larger than the length of the first length region 24a. As a result, the second non-forming portion 24e is arranged to sufficiently follow the axial direction. As a result, when the linear member 20 is inserted into the tubular member 10, the second non-forming portion 24e can slide the sealing member 30 straight in the axial direction.

In the present embodiment, a difference (hereinafter, also referred to as a clearance; the sum of the difference L2a and the difference L2b in FIG. 4) between an inner diameter (inner diameter L3 in FIG. 4) of the tubular member 10 and an amplitude (amplitude L4 in FIG. 4) of a portion (wave shape forming portion 24c) of the intermediate portion 24 having the wave shape when the linear member 20 is arranged outside the tubular member 10 (referred to as a natural state) is equal to or more than 0.095 mm and less than 0.135 mm. A force (hereinafter, referred to as a pull-out strength) required to relatively move the linear member 20 to the base end side of the tubular member 10 with respect to the tubular member 10 is equal to or more than 2.6 N and less than 6.5 N.

As described above, since the clearance is a sufficient value, the intermediate portion 24 is in pressure contact with the inner wall of the tubular member 10 at the bent portion 29, and the sliding resistance of the intermediate portion 24 with respect to the inner wall of the tubular member 10 is large. As a result, the linear member 20 is prevented from being unexpectedly pulled out from the tubular member 10. Further, since the pull-out strength is equal to or more than 2.6 N and less than 6.5 N, it is relatively easy to pull out the linear member 20 from the tubular member 10 using an instrument or the like, but it is difficult to pull out the linear member 20 from the tubular member 10 by gripping the base end portion 22 with a hand or the like, and the linear member 20 is prevented from being unexpectedly pulled out from the tubular member 10.

As described above, the inner diameter of the tubular member 10 is smaller than the amplitude of the wave shape forming portion 24c in the natural state. That is, the clearance is a value obtained by subtracting the inner diameter of the tubular member 10 from the amplitude of the wave shape forming portion 24c in the natural state.

The pull-out strength is a force required to fix the tubular member 10 and pull out the linear member 20 inserted into the tubular member 10 from the tubular member 10. The pulling out of the linear member 20 from the tubular member 10 is to move the linear member 20 relative to the tubular member 10 toward the base end side by a predetermined distance.

As described above, the outer diameter of the intermediate portion 24 is preferably smaller than a difference between the outer diameter of the leading end portion 26 and the outer diameter of the base end portion 22. As a result, the linear member 20 can be pulled out from the tubular member 10 at a predetermined pull-out strength even when the clearance is large.

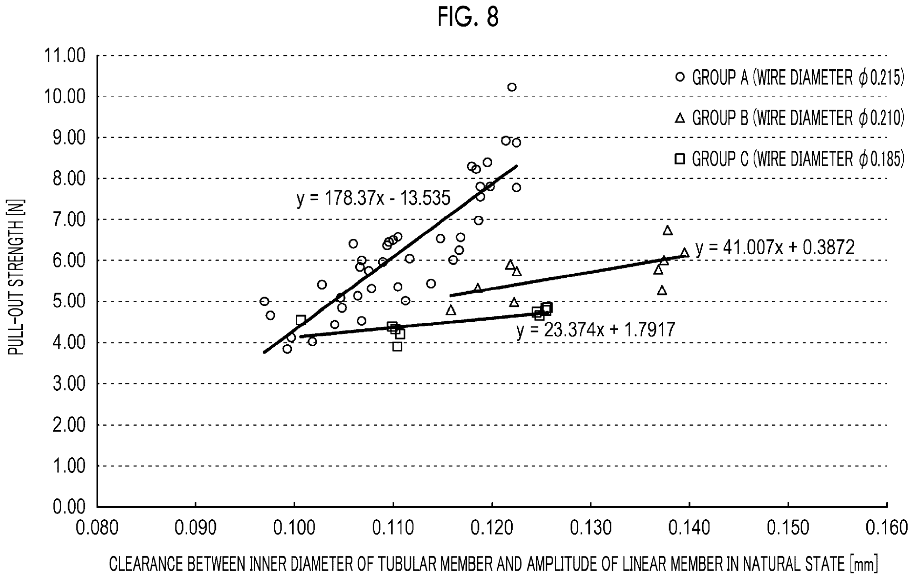

In this regard, a relationship between the clearance and the pull-out strength in a plurality of medical instruments 1 will be described with reference to FIG. 8. As shown in FIG. 8, there is a positive correlation between the clearance and the pull-out strength. FIG. 8 is a graph showing a case where the clearance of each medical instrument 1 in a medical instrument 1 group in which the outer diameter of the intermediate portion 24 is different is plotted on the x-axis, the pull-out strength is plotted on the y-axis, and the relationship of the medical instrument 1 group is approximated to a linear function. FIG. 8 is created such that the medical instrument 1 group in which the outer diameter (its average) of the intermediate portion 24 is 0.215 mm, 0.210 mm, and 0.185 mm is created as a group A, a group B, and a group C, respectively. In any of the group A, the group B, and the group C, the difference between the outer diameter of the leading end portion 26 and the outer diameter of the base end portion 22 is 0.190 mm. That is, in the group A and the group B, the outer diameter of the intermediate portion 24 is larger than the difference between the outer diameter of the leading end portion 26 and the outer diameter of the base end portion 22, and in the group C, the outer diameter of the intermediate portion 24 is smaller than the difference between the outer diameter of the leading end portion 26 and the outer diameter of the base end portion 22.

As shown in FIG. 8, when the clearance is in a range of equal to or more than 0.090 mm and less than 0.15 mm, which sufficiently includes a range of equal to or more than 0.095 mm and less than 0.135 mm, the pull-out strength in the medical instrument 1 of the group A takes a value of 3.5 N to 10.5 N as an actual measured value, and the pull-out strength in the medical instrument 1 of the group B takes a value of 4.5 N to 7.0 N as an actual measured value. On the other hand, in the medical instrument 1 of the group C, the pull-out strength takes a value of 3.5 N to 5.0 N as an actual measured value. That is, among the medical instruments 1 of the group A to the group C, only in the medical instrument 1 of the group C, the value of the pull-out strength is within a value of 2.6 N or more and less than 6.5 N. When the clearance is in a range of equal to or more than 0.095 mm and less than 0.135 mm, in the medical instrument 1 group of the group B and the group C, the actual measured value of the value of the pull-out strength is within a value of equal to or more than 2.6 N and less than 6.5 N.

When the clearance is in a range of equal to or more than 0.090 mm and less than 0.15 mm, the pull-out strength calculated based on the approximate expression of the graph is as follows. The pull-out strength in the group A is equal to or more than 2.5 N and equal to or less than 13.3 N, and the pull-out strength in the group B is equal to or more than 4.0 N and equal to or less than 6.6 N. On the other hand, the pull-out strength in the group C is equal to or more than 3.8 N and equal to or less than 5.4 N, and the pull-out strength in the group C is within the above range of equal to or more than 2.6 N and less than 6.5 N. When the clearance is in a range of equal to or more than 0.095 mm and less than 0.135 mm, the value of the pull-out strength in the medical instrument 1 of the group B and the group C is within the above range of equal to or more than 2.6 N and less than 6.5 N.

The slopes of the approximate expressions of the graphs in the group A, the group B, and the group C are 178.37 [N/mm], 41.01 [N/mm], and 23.37 [N/mm], respectively, and the intercepts are -13.54 N, 0.39 N, and 1.79 N, respectively. In the medical instrument 1 group consisting of the plurality of medical instruments 1, a slope when a relationship between an increase amount of the pull-out strength and an increase amount of the clearance is approximated to a linear function when the clearance [mm] is taken on the X-axis and the pull-out strength [N] is taken on the Y-axis is equal to or more than 10, preferably equal to or more than 20. The slope is less than 100, preferably less than 50, and more preferably less than 30. By setting the slope to a value smaller than a predetermined value, the range of the pull-out strength in the medical instrument 1 can be reduced even when the range of the clearance is large. On the other hand, when the slope is larger than the predetermined value, the linear member 20 has sufficient rigidity (in order to have a sufficient pull-out strength) because the linear member 20 is unlikely to be pulled out from the tubular member 10.

Next, the leading end portion (a portion in which the balloon member 40 is arranged and a vicinity thereof) of the tubular member 10 will be described in detail.

In the present embodiment, when a liquid or a gas (referred to as a liquid or the like) is injected through the injection port 18 to increase the volume of the balloon member 40, the balloon member 40 is expanded by increasing the dimensions of the balloon member 40 in the radial direction and the axial direction of the tubular member 10.

As shown in FIG. 5, the attachment portion 13 is a portion of the tubular member 10 arranged on the inner diameter side (particularly, inside the hollow portion 42) of the balloon member 40. Specifically, the attachment portion 13 is a partial length region of the tube wall of the tubular member. The attachment portion 13 includes the spring forming portion 15 on the base end side and a spring non-forming portion 17 on the leading end side. The spring forming portion 15 is a portion in which a spring that is expandable and contractible in the axial direction is formed by forming the helical slit 44 or the like. The spring non-forming portion 17 is a portion in which the helical slit 44 is not formed and in which a spring that does not substantially expand or contract in the axial direction is not formed.

Since a portion of the attachment portion 13 on the leading end side does not form the spring, the portion does not have the flexibility of the spring forming portion 15 and is more rigid than the spring forming portion 15. Therefore, when the balloon member 40 is inflated and expanded in the axial direction, the balloon member 40 can be expanded straight with respect to the extending direction of the spring non-forming portion 17 that is a portion of the attachment portion 13 on the leading end side. In other words, when the entire attachment portion 13 is a spring when the balloon member 40 is expanded in the axial direction, the entire attachment portion 13 may have flexibility, and the balloon member 40 may be expanded while being unexpectedly bent or twisted. However, since the portion of the attachment portion 13 on the leading end side does not have large flexibility, the balloon member 40 can be expanded approximately in the axial direction.

In the present embodiment, a central portion of the attachment portion 13 in the axial direction is the spring forming portion 15. In other words, a length (length in the axial direction) of the spring forming portion 15 in the attachment portion 13 is larger than a length (length in the axial direction) of the spring non-forming portion 17 in the attachment portion 13.

Instead of the present embodiment, substantially the entire attachment portion 13 in the axial direction may be the spring forming portion 15. In addition, only the central portion of the attachment portion 13 in the axial direction may be the spring forming portion 15, and both end portions of the attachment portion 13 in the axial direction may be the spring non-forming portion 17.

The winding pitch in the second region 15a2 in the spring is smaller than the winding pitch in the first region 15a1 in the spring. In addition, the winding pitch in the third region 15a3 in the spring is larger than the winding pitch in the second region 15a2. Here, the winding pitch is a distance in the axial direction from one end on the leading end side of one turn to one end on the leading end side of another turn adjacent to the leading end side of the one turn. That is, the winding pitch can also be said to be a value that is the sum of the width dimension of the helical slit 44 and the width dimension of the ribbon 15a. In addition, in the present embodiment, the width of the helical slit 44 in the reduced-diameter state is substantially constant from the first region 15a1 to the third region 15a3. That is, the width of the ribbon 15a in the second region 15a2 in the spring is smaller than the width of the ribbon 15a in the first region 15a1 in the spring. Further, the width of the ribbon 15a in the third region 15a3 in the spring is larger than the width of the ribbon 15a in the second region 15a2.

The first region 15a1 is a predetermined length region on the base end side in the spring. The second region 15a2 is a length region on the leading end side with respect to the first region 15a1 in the spring. The third region 15a3 is a length region on the leading end side with respect to the second region 15a2 in the spring. In the present embodiment, the winding pitch in the second region 15a2 is the minimum value among the winding pitches of the attachment portion 13.

Since the winding pitch is small in the second region 15a2, the amount of the liquid flowing into the hollow portion 42 is increased, and the liquid can be favorably fed to the hollow portion 42. In addition, since the winding pitch in the third region 15a3 on the leading end side of the second region 15a2 is large, the tubular member 10 is prevented from being unexpectedly bent between the spring forming portion 15 and the spring non-forming portion 17.

In the present embodiment, the first region 15a1 to the third region 15a3 are all arranged in the attachment portion 13. The first region 15a1 to the third region 15a3 are particularly arranged inside the hollow portion 42. As described above, since the first region 15a1 and the third region 15a3, which do not have the minimum winding pitch, are arranged at both end portions of the attachment portion 13, the attachment portion 13 is prevented from having excessive flexibility.

In the large-diameter state or the small-diameter state, the width of the helical slit 44 in the second region 15a2 is preferably larger than the width of the helical slit 44 in the first region 15a1 or the third region 15a3. In this way, since the width of the helical slit 44 in the second region 15a2 is large, the hollow portion 42 and the lumen 11 can be sufficiently communicated with each other. In addition, since the width of the helical slit 44 in the first region 15a1 or the third region 15a3 is small, the first region 15a1 or the third region 15a3 is maintained to be rigid, and the attachment portion 13 can be prevented from having excessive flexibility. That is, with the above configuration, the hollow portion 42 and the lumen 11 can sufficiently communicate with each other in the second region 15a2 while the rigidity of the attachment portion 13 can be maintained within a desired range.

The large-diameter state and the small-diameter state each refer to a state in which the balloon member 40 is expanded. When the balloon member 40 is expanded from the reduced-diameter state, the balloon member 40 reaches the small-diameter state, and then reaches the large-diameter state. The large-diameter state is a state in which the dimension of the balloon member 40 in the width direction is increased to the same degree as the generally assumed maximum inner diameter that can be assumed for the body lumen to be used with the medical instrument 1. For example, in the medical instrument 1 according to the present embodiment, the balloon member 40 is arranged in the blood vessel (the carotid artery is an example), and an example of the general inner diameter of the blood vessel is 5 mm, and an example of the generally assumed large inner diameter of the carotid artery is 6 mm to 7 mm. That is, the large-diameter state of the balloon member 40 in the medical instrument 1 according to the present embodiment is a state in which the balloon member 40 is expanded until the dimension of the balloon member 40 in the width direction is about 6.5 mm. Here, the amount of fluid that needs to be pressure-injected from the reduced-diameter state to the large-diameter state is referred to as a total pressure-injection amount. The small-diameter state is a state of the balloon member 40 when an amount of fluid of about one-fourth of the total pressure-injection amount is injected.

In the present embodiment, the spring is also formed in a portion (base end side forming portion 15d) of the tube wall on the base end side with respect to the attachment portion 13. That is, the base end side forming portion 15d is also a portion of the spring forming portion 15. More specifically, the spring is formed by a common helical slit 44 not only in the tube wall inside the hollow portion 42 but also in the tube wall arranged on the inner diameter side of the seal band 46 and the first adhesive 51 and the tube wall (tube wall in the base end side forming portion 15d) on the base end side with respect to the first adhesive 51. That is, the spring is integrally formed in the tube wall inside the hollow portion 42, the tube wall arranged on the inner diameter side of the seal band 46 and the first adhesive 51, and the tube wall in the base end side forming portion 15d.

The spring is also formed in the base end side forming portion 15d, and thus the base end side forming portion 15d has flexibility. As a result, when the balloon member 40 is arranged in the body lumen, the tubular member 10 in the vicinity of the balloon member 40 can follow the shape of the body lumen, and unnecessary load is prevented from being applied to the balloon member 40 and the member (for example, the adhesive 50) in the vicinity thereof. Therefore, the deformation of the balloon member 40 and the member in the vicinity thereof is suppressed, and, for example, the damage to the balloon member 40 or the liquid leakage from the balloon member 40 is suppressed.

The winding pitch of the spring formed in the base end side forming portion 15d is larger than the winding pitch in the third region 15a3. In other words, the width of the ribbon 15a in the base end side forming portion 15d is larger than the width of the ribbon 15a in the third region 15a3. As a result, the tubular member 10 is prevented from being easily bent between the spring forming portion 15 and the spring non-forming portion 17 on the base end side of the spring forming portion 15.

In the present embodiment, an angle of a portion of the ribbon 15a in the helical direction with respect to the axial direction in the reduced-diameter state is equal to or more than 60 degrees. More specifically, in the second region 15a2, the angle of the portion of the ribbon 15a in the helical direction with respect to the axial direction need only be equal to or more than 60 degrees. Instead of this, an angle of substantially the entire ribbon 15a in the attachment portion 13 in the helical direction with respect to the axial direction may be equal to or more than 60 degrees. Alternatively, the angle is more preferably equal to or more than 75 degrees. Therefore, the expansion and contraction of the spring forming portion 15 in the axial direction is more favorable. As a result, the balloon member 40 can be sufficiently expanded in the axial direction in the small-diameter state or the large-diameter state (expanded state).

In the small-diameter state or the large-diameter state, the angle of the portion of the ribbon 15a in the helical direction with respect to the axial direction may be equal to or more than 60 degrees in the same manner.

The present invention is not limited to the above embodiments, and various aspects of modifications, improvements, and the like are also included as long as the object of the present invention is achieved.

For example, in the above embodiments, the carotid artery is an example of the blood vessel in which the balloon-equipped guide wire 1 is arranged, but the blood vessel is not limited to this example, and may be another blood vessel such as the aorta. In this case, since the inner diameter is different depending on the blood vessel, the dimension of the balloon member 40 can be changed as appropriate. In addition, the balloon-equipped guide wire 1 may be used for post-expansion of a stent graft (not shown) or the like arranged in another blood vessel (for example, the aorta), and the dimension of the balloon member 40 can be changed as appropriate according to the inner diameter of the stent graft. Specifically, when the balloon-equipped guide wire 1 is arranged in the aorta or when the balloon-equipped guide wire 1 is used for post-expansion of the stent graft, the dimension of the balloon member 40 in the width direction in the large-diameter state is about 20 mm to 40 mm.

The above embodiments include the following technical idea.
(1) A balloon-equipped guide wire including: a tubular member that has a lumen and is provided with an opening through which the lumen communicates with an exterior space in a tube wall at a base end portion of the tubular member; a balloon member that is provided on an outer diameter side of a leading end portion of the tubular member, has an inner portion that communicates with the lumen of the tubular member, and is expandable by increasing a volume of the inner portion; and a linear member that is arranged in the lumen at the base end portion of the tubular member and is movable in an axial direction of the tubular member, in which a sealing member that is in close contact with a portion of an inner wall of the tubular member is provided at a leading end portion of the linear member, the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening, the tube wall at the leading end portion of the tubular member is a spring in which a ribbon is helically arranged with a gap between turns with a central axis of the tubular member as an axis, a width dimension of the gap is less than 1/5 of a width dimension of the ribbon, and when a liquid or a gas is injected through the opening to increase the volume of the balloon member, the balloon member is expanded by increasing dimensions of the balloon member in both a radial direction and the axial direction of the tubular member.
(2) The balloon-equipped guide wire according to (1), in which an attachment portion that is a portion of the tube wall arranged on an inner diameter side of the balloon member in the tubular member includes a spring forming portion in which the spring is formed, on the base end side, and a spring non-forming portion in which the spring is not formed, on the leading end side.
(2-1) The balloon-equipped guide wire, in which a length of the spring forming portion in the axial direction in the attachment portion is larger than a length of the spring non-forming portion in the axial direction in the attachment portion.
(3) The balloon-equipped guide wire according to (2), in which the inner portion of the balloon member and the lumen communicate with each other through the gap, a winding pitch in a second region on the leading end side with respect to a first region on the base end side in the spring is smaller than a winding pitch in the first region, and a winding pitch in a third region on the leading end side with respect to the second region in the spring is larger than the winding pitch in the second region.
(3-2) The balloon-equipped guide wire, in which the first region, the second region, and the third region are arranged in an attachment portion inside a hollow portion.
(3-3) The balloon-equipped guide wire, in which a width of a helical slit in the second region is larger than a width of a helical slit 44 in the first region or the third region in a large-diameter state or a small-diameter state.
(4) The balloon-equipped guide wire according to (3), in which the spring is also formed in a portion of the tube wall on the base end side with respect to the attachment portion.
(4-1) The balloon-equipped guide wire, in which a winding pitch of a spring formed in a base end side forming portion is larger than the winding pitch in the third region.
(5) The balloon-equipped guide wire according to any one of (1) to (4), in which, in an initial stage of expansion of the balloon member, an expansion rate of the balloon member in the axial direction is equal to or more than 5.0% of an expansion rate of the balloon member in the radial direction.
(6) The balloon-equipped guide wire according to (5), in which an angle of a portion of the ribbon in a helical direction of the ribbon with respect to the axial direction is equal to or more than 60 degrees.
(6-1) The balloon-equipped guide wire, in which the angle of the portion of the ribbon in the helical direction with respect to the axial direction is equal to or more than 75 degrees.
(7) A catheter set including: a balloon-equipped guide wire; and a catheter into which the balloon-equipped guide wire is inserted, in which the balloon-equipped guide wire includes a tubular member that has a lumen and is provided with an opening through which the lumen communicates with an exterior space in a tube wall at a base end portion of the tubular member, a balloon member that is provided on an outer diameter side of a leading end portion of the tubular member, has an inner portion that communicates with the lumen of the tubular member, and is expandable by increasing a volume of the inner portion, and a linear member that is arranged in the lumen at the base end portion of the tubular member and is movable in an axial direction of the tubular member, a sealing member that is in close contact with a portion of an inner wall of the tubular member is provided at a leading end portion of the linear member, the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening, the tube wall at the leading end portion of the tubular member is a spring in which a ribbon is helically arranged with a gap between turns with a central axis of the tubular member as an axis, a width dimension of the gap is less than 1/5 of a width dimension of the ribbon, and when a liquid or a gas is injected through the opening to increase the volume of the balloon member, the balloon member is expanded by increasing dimensions of the balloon member in both a radial direction and the axial direction of the tubular member.
(7-1) The catheter set, in which a width of a wire lumen into which the balloon-equipped guide wire is inserted is smaller than the width of the balloon member in a reduced-diameter state or an expanded state.
(8) A medical instrument including: a tubular member; and a linear member that is arranged in a lumen at a base end portion of the tubular member and is movable in an axial direction of the tubular member, in which a sealing member that is in close contact with a portion of an inner wall that defines the lumen of the tubular member is provided at a leading end portion of the linear member, the linear member includes the leading end portion, an intermediate portion on a base end side with respect to the leading end portion, and a base end portion on the base end side with respect to the intermediate portion, the leading end portion, the intermediate portion, and the base end portion each have a partial length region having a uniform outer diameter, the outer diameter of the partial length region of the intermediate portion is smaller than 85% of a width of the lumen of the tubular member, and among the partial length regions of the leading end portion, the intermediate portion, and the base end portion, the partial length region of the base end portion is the thickest, and the partial length region of the leading end portion is the thinnest.
(8-1) The medical instrument, in which the outer diameter of the intermediate portion is smaller than a difference between an outer diameter of the leading end portion and an outer diameter of the base end portion.
(9) The medical instrument according to (8), in which a first transition portion between the leading end portion and the intermediate portion and a second transition portion between the intermediate portion and the base end portion each have a tapered shape, an angle formed by the tapered shape of the second transition portion with respect to the axial direction is larger than an angle formed by the tapered shape of the first transition portion with respect to the axial direction, and a wire diameter of the base end portion is larger than the width of the lumen.
(9-1) The medical instrument, in which a length of the first transition portion in the axial direction is smaller than a length of the second transition portion in the axial direction.
(10) The medical instrument according to (9), in which the intermediate portion of the linear member is bent at a plurality of bent portions including first bent portions and second bent portions to have a wave shape, the wave shape is formed by being bent upward or bent downward at each of a plurality of the first bent portions and a plurality of the second bent portions that are alternately arranged, a length of a first length region from one of the first bent portions to one of the second bent portions adjacent to the one of the first bent portions on a leading end side with respect to the one of the first bent portions is smaller than a length of a second length region from the one of the second bent portions to another of the first bent portions adjacent to the one of the second bent portions on a further leading end side with respect to the one of the second bent portions, and an angle formed by the first length region with respect to the axial direction is larger than an angle formed by the second length region with respect to the axial direction.
(10-1) The medical instrument, in which a length of one first length region is larger than a length of another first length region located on the leading end side with respect to the one first length region, and a length of one second length region is larger than a length of another second length region located on the leading end side with respect to the one second length region.
(11) The medical instrument according to (10), in which, in the intermediate portion, an amplitude of the wave shape in a portion on the leading end side is smaller than an amplitude of the wave shape in another portion on the base end side with respect to the portion on the leading end side.
(11-1) The medical instrument, in which an amplitude in a partial length region including a first bent portion and a second bent portion that are arranged on a most leading end side in the intermediate portion is smaller than an average amplitude of a length region in the vicinity of a group of the first bent portion and the second bent portion that are close to each other.
(12) The medical instrument according to (11), in which a radius of curvature of the bent portion is smaller than the length of the second length region.
(12-1) The medical instrument, in which the radius of curvature of the bent portion is larger than the length of the first length region.
(13) The medical instrument according to (12), in which the intermediate portion includes a wave shape forming portion in which the wave shape is formed, a first wave shape non-forming portion that is a wave shape non-forming portion in which the wave shape is not formed, and is arranged on the base end side with respect to the wave shape forming portion, and a second wave shape non-forming portion that is a wave shape non-forming portion in which the wave shape is not formed, and is arranged on the leading end side with respect to the wave shape forming portion, and a length of the first wave shape non-forming portion in the axial direction is smaller than a length of the second wave shape non-forming portion in the axial direction.
(13-1) The medical instrument, in which a length of the first non-forming portion in the axial direction is smaller than a length of the wave shape forming portion in the axial direction.
(13-2) The medical instrument, in which a length of the second non-forming portion in the axial direction is larger than the length of the first length region.
(14) The medical instrument according to any one of (10) to (13), in which when the linear member is arranged outside the tubular member, a difference between an inner diameter of the tubular member and an amplitude of a portion of the linear member having the wave shape of the intermediate portion is equal to or more than 0.095 mm and less than 0.135 mm, and a force required to move the linear member relative to the tubular member toward the base end side of the tubular member is equal to or more than 2.6 N and less than 6.5 N.
(15) The medical instrument according to (14), in which a radius of curvature of the one of the first bent portions is smaller than a radius of curvature of the one of the second bent portions adjacent to the one of the first bent portions across the first length region.
(15-1) The medical instrument, in which a radius of curvature of the first bent portion arranged on the leading end side is larger than a radius of curvature of another first bent portion arranged on the base end side with respect to the first bent portion, and a radius of curvature of the second bent portion arranged on the leading end side is larger than a radius of curvature of another second bent portion arranged on the base end side with respect to the second bent portion.
(16) A medical instrument group, in which a slope when a relationship between an increase amount of a pull-out strength and an increase amount of the difference between the inner diameter of the tubular member and the amplitude is approximated to a linear function when the difference [mm] is taken on an X-axis and the pull-out strength [N] is taken on a Y-axis is equal to or more than 10 and preferably equal to or more than 20, and the slope is less than 100, preferably less than 50, and more preferably less than 30.
(17) A balloon-equipped guide wire, in which an expansion rate in a width direction decreases as an expansion stage progresses, and an expansion rate in an axial direction increases as the expansion stage progresses.

This application claims priority based on Japanese Patent Application No. 2023-165927 filed on September 27, 2023 and Japanese Patent Application No. 2023-165928 filed on September 27, 2023, the entire disclosures of which are incorporated herein by reference.

### REFERENCE SIGNS LIST

1 Medical instrument, Balloon-equipped guide wire
10 Tubular member
11 Lumen
12 Base end opening portion
13 Attachment portion
14 Coil portion
15 Spring forming portion
15a Ribbon
15a1 First region
15a2 Second region
15a3 Third region
15d Base end side forming portion
16 Leading end tip
17 Spring non-forming portion
18 Injection port
19 Radiopaque marker
20 Linear member
22 Base end portion
23 Second transition portion
24 Intermediate portion
24a First length region
24b Second length region
24c Wave shape forming portion
24d First non-forming portion
24e Second non-forming portion
25 First transition portion
26 Leading end portion
29 Bent portion
29a, 29a1, 29a2 First bent portion
29b, 29b1 Second bent portion
30 Sealing member
40 Balloon member
41 Film portion
42 Hollow portion
44 Helical slit
46 Seal band
50 Adhesive
51 First adhesive
52 Second adhesive
53 Third adhesive
100 Catheter set
200 Catheter
210 Wire lumen
220 Main lumen

## Claims

1. A balloon-equipped guide wire comprising:
a tubular member that has a lumen and is provided with an opening through which the lumen communicates with an exterior space in a tube wall at a base end portion of the tubular member;
a balloon member that is provided on an outer diameter side of a leading end portion of the tubular member, has an inner portion that communicates with the lumen of the tubular member, and is expandable by increasing a volume of the inner portion; and
a linear member that is arranged in the lumen at the base end portion of the tubular member and is movable in an axial direction of the tubular member,
wherein a sealing member that is in close contact with a portion of an inner wall of the tubular member is provided at a leading end portion of the linear member,
the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening,
the tube wall at the leading end portion of the tubular member is a spring in which a ribbon is helically arranged with a gap between turns with a central axis of the tubular member as an axis,
a width dimension of the gap is less than 1/5 of a width dimension of the ribbon, and
when a liquid or a gas is injected through the opening to increase the volume of the balloon member, the balloon member is expanded by increasing dimensions of the balloon member in both a radial direction and the axial direction of the tubular member.

2. The balloon-equipped guide wire according to Claim 1,
wherein an attachment portion that is a portion of the tube wall arranged on an inner diameter side of the balloon member in the tubular member includes a spring forming portion in which the spring is formed, on the base end side, and a spring non-forming portion in which the spring is not formed, on the leading end side.

3. The balloon-equipped guide wire according to Claim 2,
wherein the inner portion of the balloon member and the lumen communicate with each other through the gap,
a winding pitch in a second region on the leading end side with respect to a first region on the base end side in the spring is smaller than a winding pitch in the first region, and
a winding pitch in a third region on the leading end side with respect to the second region in the spring is larger than the winding pitch in the second region.

4. The balloon-equipped guide wire according to Claim 3,
wherein the spring is also formed in a portion of the tube wall on the base end side with respect to the attachment portion.

5. The balloon-equipped guide wire according to any one of Claims 1 to 4,
wherein, in an initial stage of expansion of the balloon member, an expansion rate of the balloon member in the axial direction is equal to or more than 5.0% of an expansion rate of the balloon member in the radial direction.

6. The balloon-equipped guide wire according to Claim 5,
wherein an angle of a portion of the ribbon in a helical direction of the ribbon with respect to the axial direction is equal to or more than 60 degrees.

7. A catheter set comprising:
a balloon-equipped guide wire; and
a catheter into which the balloon-equipped guide wire is inserted,
wherein the balloon-equipped guide wire includes
a tubular member that has a lumen and is provided with an opening through which the lumen communicates with an exterior space in a tube wall at a base end portion of the tubular member,
a balloon member that is provided on an outer diameter side of a leading end portion of the tubular member, has an inner portion that communicates with the lumen of the tubular member, and is expandable by increasing a volume of the inner portion, and
a linear member that is arranged in the lumen at the base end portion of the tubular member and is movable in an axial direction of the tubular member,
a sealing member that is in close contact with a portion of an inner wall of the tubular member is provided at a leading end portion of the linear member,
the sealing member moves, with movement of the linear member in the axial direction, between a closed position at which the sealing member closes the opening or is arranged on a leading end side with respect to the opening to block a flow of a fluid from the opening toward the inner portion and an open position on a base end side of the tubular member with respect to the opening,
the tube wall at the leading end portion of the tubular member is a spring in which a ribbon is helically arranged with a gap between turns with a central axis of the tubular member as an axis,
a width dimension of the gap is less than 1/5 of a width dimension of the ribbon, and
when a liquid or a gas is injected through the opening to increase the volume of the balloon member, the balloon member is expanded by increasing dimensions of the balloon member in both a radial direction and the axial direction of the tubular member.

8. A medical instrument comprising:
a tubular member; and
a linear member that is arranged in a lumen at a base end portion of the tubular member and is movable in an axial direction of the tubular member,
wherein a sealing member that is in close contact with a portion of an inner wall that defines the lumen of the tubular member is provided at a leading end portion of the linear member,
the linear member includes the leading end portion, an intermediate portion on a base end side with respect to the leading end portion, and a base end portion on the base end side with respect to the intermediate portion,
the leading end portion, the intermediate portion, and the base end portion each have a partial length region having a uniform outer diameter,
the outer diameter of the partial length region of the intermediate portion is smaller than 85% of a width of the lumen of the tubular member, and
among the partial length regions of the leading end portion, the intermediate portion, and the base end portion, the partial length region of the base end portion is the thickest, and the partial length region of the leading end portion is the thinnest.

9. The medical instrument according to Claim 8,
wherein a first transition portion between the leading end portion and the intermediate portion and a second transition portion between the intermediate portion and the base end portion each have a tapered shape,
an angle formed by the tapered shape of the second transition portion with respect to the axial direction is larger than an angle formed by the tapered shape of the first transition portion with respect to the axial direction, and
a wire diameter of the base end portion is larger than the width of the lumen.

10. The medical instrument according to Claim 9,
wherein the intermediate portion of the linear member is bent at a plurality of bent portions including first bent portions and second bent portions to have a wave shape,
the wave shape is formed by being bent upward or bent downward at each of a plurality of the first bent portions and a plurality of the second bent portions that are alternately arranged,
a length of a first length region from one of the first bent portions to one of the second bent portions adjacent to the one of the first bent portions on a leading end side with respect to the one of the first bent portions is smaller than a length of a second length region from the one of the second bent portions to another of the first bent portions adjacent to the one of the second bent portions on a further leading end side with respect to the one of the second bent portions, and
an angle formed by the first length region with respect to the axial direction is larger than an angle formed by the second length region with respect to the axial direction.

11. The medical instrument according to Claim 10,
wherein, in the intermediate portion, an amplitude of the wave shape in a portion on the leading end side is smaller than an amplitude of the wave shape in another portion on the base end side with respect to the portion on the leading end side.

12. The medical instrument according to Claim 11,
wherein a radius of curvature of the bent portion is smaller than the length of the second length region.

13. The medical instrument according to Claim 12,
wherein the intermediate portion includes a wave shape forming portion in which the wave shape is formed, a first wave shape non-forming portion that is a wave shape non-forming portion in which the wave shape is not formed, and is arranged on the base end side with respect to the wave shape forming portion, and a second wave shape non-forming portion that is a wave shape non-forming portion in which the wave shape is not formed, and is arranged on the leading end side with respect to the wave shape forming portion, and
a length of the first wave shape non-forming portion in the axial direction is smaller than a length of the second wave shape non-forming portion in the axial direction.

14. The medical instrument according to any one of Claims 10 to 13,
wherein ,when the linear member is arranged outside the tubular member, a difference between an inner diameter of the tubular member and an amplitude of a portion of the linear member having the wave shape of the intermediate portion is equal to or more than 0.095 mm and less than 0.135 mm, and
a force required to move the linear member relative to the tubular member toward the base end side of the tubular member is equal to or more than 2.6 N and less than 6.5 N.

15. The medical instrument according to Claim 14,
wherein a radius of curvature of one of the first bent portions is smaller than a radius of curvature of one of the second bent portions adjacent to the one of the first bent portions across the first length region.
